# EUROPEAN PATENT SPECIFICATION

(11) **EP 0 979 286 B1**
(45) Date of publication and mention of the grant of the patent: **29.01.2014**
(21) Application number: 98918891.7
(22) Date of filing: 30.04.1998
(51) Int. Cl.: C12N 15/52, C12N 15/76, C12N 9/00, C12N 1/21, C12P 17/06, C07D 309/00, C07D 313/04, C07D 493/10, C07D 313/00, C07H 17/04

(54) **COMBINATORIAL POLYKETIDE LIBRARIES PRODUCED USING A MODULAR PKS GENE CLUSTER AS SCAFFOLD**
POLYKETIDEKOMBINATIONSBIBLIOTHEKEN HERGESTELLT MIT MODULAREM PKS GENCLUSTER ALS GERÜST
BIBLIOTHEQUES COMBINATOIRES DE POLYCETIDES PRODUITES A L'AIDE D'UN GROUPE DE GENES MODULAIRE DE POLYCETIDE SYNTHASE EN TANT QUE SQUELETTE

(30) Priority: 30.04.1997 US 846247; 05.03.1998 US 76919 P
(43) Date of publication of application: 16.02.2000
(73) Proprietor: Kosan Biosciences, Burlingame, CA 94010 (US); The Board of Trustees of the Leland Stanford Junior University, Palo Alto, CA 94306-1106 (US)
(72) Inventor: KHOSLA, Chaitan, Palo Alto, CA 94306 (US); ASHLEY, Gary, Alameda, CA 94502 (US); FU, Hong, Fremont, CA 94555 (US); KAO, Camilla, M., Palo Alto, CA 94306 (US); MCDANIEL, Robert, Palo Alto, CA 94306 (US)
(74) Representative: Gibson, Mark
(86) International application number: PCT/US1998/008792
(87) International publication number: WO 1998/049315

(56) References cited:
- WO-A1-96/40968
- KHOSLA C ET AL: "Generation of polyketide libraries via combinatorial biosynthesis." TRENDS BIOTECHNOL, SEP 1996, 14 (9) P335-41, ENGLAND, XP004035738
- KAO CM ET AL: "Engineered biosynthesis of a complete macrolactone in a heterologous host." SCIENCE, JUL 22 1994, 265 (5171) P509-12, UNITED STATES, XP002073974

## Description

### Reference to Government Funding

This work was supported in part by a grant from the National Institutes of Health, CA66736. The U.S. government has certain rights in this invention.

### Technical Field

The invention relates to the field of combinatorial libraries, to novel polyketides and antibiotics and to methods to prepare them. More particularly, it concerns construction of new polyketides and to libraries of polyketides synthesized by polyketide synthases derived from a naturally occurring PKS, as illustrated by the erythromycin gene cluster.

### Background Art

Polyketides represent a large family of diverse compounds ultimately synthesized from 2-carbon units through a series of Claisen-type condensations and subsequent modifications. Members of this group include antibiotics such as tetracyclines, anticancer agents such as daunomycin, and immunosuppressants such as FK506 and rapamycin. Polyketides occur in many types of organisms including fungi and mycelial bacteria, in particular, the actinomycetes.

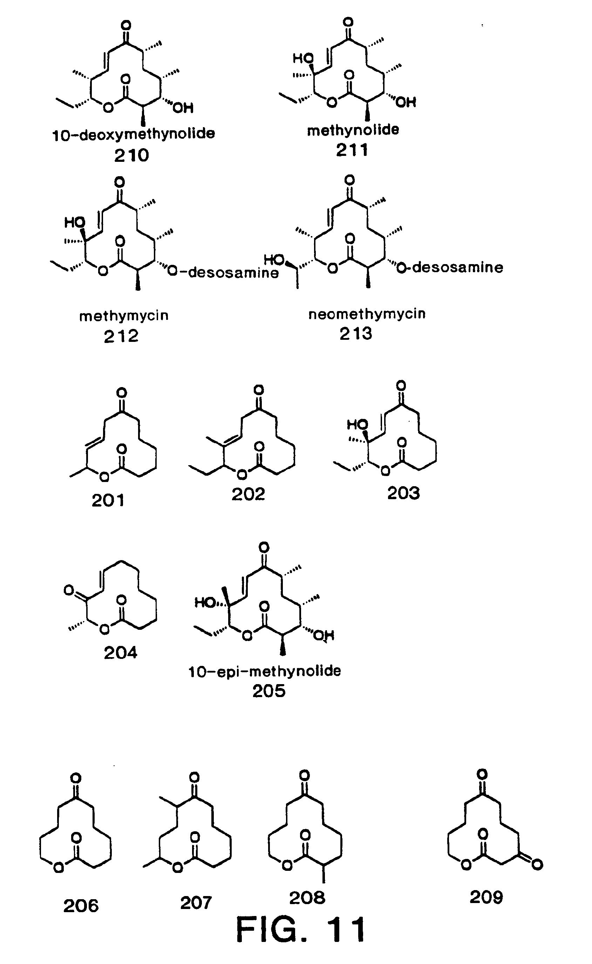

A number of lactones of keto acids have been synthesized using standard organic chemistry. These include a series of unsaturated ketolactones synthesized by Vedejes et al., J. Am Chem Soc (1987) 109:5437-5446, shown as formulas 201, 202 and 203 in Figure 11 herein. Additional compounds of formulas 204 and 205, also shown in Figure 11 were synthesized as reported by Vedejes et al. J Am Chem Soc (1989) 111:8430-8438. In addition, compounds 206-208 (Figure 11) were synthesized by Borowitz J Heterocyclic Chem (1975) 12(1):101-106; compound 209 has been synthesized by Ireland et al., J Org Chem (1980) 45:1868-1880.

The polyketides are synthesized *in vivo* by polyketide synthases (PKS). This group of enzymatically active proteins is considered in a different category from the fatty acid synthases which also catalyze condensation of 2-carbon units to result in, for example, fatty acids and prostaglandins. Two major types of PKS are known which are vastly different in their construction and mode of synthesis. These are commonly referred to as Type I or "modular" and Type II, "aromatic".

The PKS scaffold that is the subject of the present invention is a member of the group designated Type I or "modular" PKS. In this type, a set of separate active sites exists for each step of carbon chain assembly and modification, but the individual proteins contain a multiplicity of such separate active sites. There may be only one multifunctional protein of this type, such as that required for the biosynthesis of 6-methyl salicylic acid (Beck, J. et al., Eur J Biochem (1990) 192:487-498; Davis, R. et al., Abstracts of Genetics of Industrial Microorganism Meeting, Montreal, Abstract P288 (1994)). More commonly, and in bacterial-derived Type I PKS assemblies, there are several such multifunctional proteins assembled to result in the end product polyketide. (Cortes, J. et al, Nature (1990) 348:176; Donadio, S. et al, Science (1991) 252:675; MacNeil, D.J. et al., Gene (1992) 115:119).

A number of modular PKS genes have been cloned. U.S. Patent No. 5,252,474 describes cloning of genes encoding the synthase for avermectin; U.S. Patent No. 5,098,837 describes the cloning of genes encoding the synthase for spiramycin; European application 791,655 and European application 791,656 describe the genes encoding the synthases for tylosin and platenolide respectively.

Khosla et al (Trends Biotechnol (1996) 14: 335-41) reviews the synthesis of polyketides and polyketide libraries by combinatorial biosynthesis. WO 96/40968 describes a host vector system used to produce PKSs which catalyse the production of a variety of polyketides, and methods for the combinatorial biosynthesis of polyketide libraries.

The PKS for erythromycin, used as an illustrative system is a modular PKS. Erythromycin was originally isolated from *S*. *erythraeus* (since reclassified as *Saccharopolyspora erythrea*) which was found in a soil sample from the Phillippine archipelago. Cloning the genes was described by Donadio, S. et al., Science (1991) 252:675. The particulars have been reviewed by Perun, T.J. in Drug Action and Drug Resistance in Bacteria, Vol. 1, S. Mitsuhashi (ed.) University Park Press, Baltimore, 1977. The antibiotic occurs in various glycosylated forms, designated A, B and C during various stages of fermentation. The entire erythromycin biosynthetic gene cluster from *S*. *erythraeus* has been mapped and sequenced by Donadio et al in Industrial Microorganisms: Basic and Applied Molecular Genetics (1993) R.H. Baltz, G.D. Hegeman, and P.L. Skatrud (eds.) (Amer Soc Microbiol) and the entire PKS is an assembly of three such multifunctional proteins usually designated DEBS-1, DEBS-2, and DEBS-3, encoded by three separate genes.

Bedford et al (Chemistry & Biology (1996) 3:827-831) report the generation of a chimeric modular PKS based on a bimodular derivative of DEBS (DEBSI+TE) in which the reductive segment of module 2 was replaced with its homologue from module 3 of DEBS.

Oliynyk et al (Chemistry & Biology (1996) 3:833-839) report the generation of a hybrid modular PKS based on replacing the entire AT domain from module 1 of DEBS1-TE by the AT domain from module 2 of the rapamycin-producing PKS.

Expression of the genes encoding the PKS complex may not be sufficient to permit the production by the synthase enzymes of polyketides when the genes are transformed into host cells that do not have the required auxiliary phosphopantetheinyl transferase enzymes which posttranslationally modify the ACP domains of the PKS. Genes encoding some of these transferases are described in WO97/13845. In addition, enzymes that mediate glycosylation of the polyketides synthesized are described in WO97/23630. U.S. Serial No. 08/989,332 (US Patent No. 6,033,883) filed 11 December 1997 describes the production of polyketides in hosts that normally do not produce them by supplying appropriate phosphopantetheinyl transferase expression systems.

There have been attempts to alter the polyketide synthase pathway of modular PKS clusters. For example, European application 238,323 describes a process for enhancing production of polyketides by introducing a rate-limiting synthase gene and U.S. Patent No. 5,514,544 describes use of an activator protein for the synthase in order to enhance production. U.S. Patent Nos. 4,874,748 and 5,149,639 describe shuttle vectors that are useful in cloning modular PKS genes in general. Methods of introducing an altered gene into a microorganism chromosome are described in WO93/13663. Modification of the loading module for the DEBS-1 protein of the erythromycin-producing polyketide synthase to substitute the loading module for the avermectin-producing polyketide synthase in order to vary the starter unit was described by Marsden, Andrew F.A. et al. Science (1998) 279:199-202 and Oliynyk, M. et al. Chemistry and Biology (1996) 3:833-839. WO 98/01571, published 15 January 1998, describes manipulation of the erythromycin PKS and novel polyketides resulting from such manipulation. In addition, WO 98/0156, also published 15 January 1998 describes a hybrid modular PKS gene for varying the nature of the starter and extender units to synthesize novel polyketides.

In addition, U.S. Patent Nos. 5,063,155 and 5,168,052 describe preparation of novel antibiotics using modular PKS systems. A number of modular PKS have been cloned. See, e.g., U.S. Patent No. 5,098,837, EP 791,655, EP 791,656 and U.S. Patent No. 5,252,474.

Type II PKS, in contrast to modular PKS, include several proteins, each of which is simpler than those found in Type I polyketide synthases. The active sites in these enzymes are used iteratively so that the proteins themselves are generally monofunctional or bifunctional. For example, the aromatic PKS complexes derived from *Streptomyces* have so far been found to contain three proteins encoded in three open reading frames. One protein provides ketosynthase (KS) and acyltransferase (AT) activities, a second provides a chain length determining factor (CLDF) and a third is an acyl carrier protein (ACP).

The present invention is concerned with PKS systems derived from modular PKS gene clusters. The nature of these clusters and their manipulation are further described below.

### Disclosure of the Invention

The invention provides recombinant materials for the production of combinatorial libraries of polyketides wherein the polyketide members of the library are synthesized by various PKS systems derived from naturally occurring PKS systems by using these systems as scaffolds. Generally, many members of these libraries may themselves be novel compounds. The invention methods may thus be directed to the preparation of an individual polyketide. The polyketide may or may not be novel, but the method of preparation permits a more convenient method of preparing it. The resulting polyketides may be further modified to convert them to antibiotics, typically through glycosylation. Also described herein are methods to recover novel polyketides with desired binding activities by screening the libraries of the invention.

Thus, in one aspect, the invention is directed to a method to prepare a nucleic acid which contains a nucleotide sequence encoding a modified polyketide synthase. A nucleic acid may be obtained by a method comprising using a naturally occurring PKS encoding sequence as a scaffold and modifying the portions of the nucleotide sequence that encode enzymatic activities, either by mutagenesis, inactivation, or replacement.

In particular, the invention provides an isolated nucleic acid molecule that encodes a non-naturally occurring modular polyketide synthase (PKS), which nucleic acid molecule is obtainable by replacing, in a nucleic acid encoding a naturally occurring PKS,
(a) the sequence encoding at least one acyl transferase catalytic region (AT) in the nucleic acid encoding said naturally occurring PKS by a sequence encoding an AT that employs a different extender unit from that employed by the AT in the naturally occurring PKS; and
(b) the sequence encoding at least one reductive cycle domain (RCD) in the nucleic acid encoding said naturally occurring PKS by a sequence encoding an RCD that contains an additional RCD catalytic activity as compared to the RCD in the naturally occurring PKS;
   wherein said RCD catalytic activity is selected from keto reductase (KR), dehydratase (DH) and enoyl reductase (ER); and wherein
   (i) said naturally occurring RCD comprises no RCD catalytic activity and said additional catalytic activity is KR, or KR plus DH, or KR plus DH plus ER; or
   (ii) said naturally occurring RCD comprises only KR and said added catalytic activity is DH or DH plus ER; or
   (iii) said naturally occurring RCD comprises only KR plus DH and said added catalytic activity is ER.

The thus modified nucleotide sequence encoding a PKS can then be used to modify a suitable host cell and the cell thus modified employed to produce a polyketide different from that produced by the PKS whose scaffolding has been used to support modifications of enzymatic activity. Also described herein are the polyketides thus produced and the antibiotics to which they may then be converted.

Further described herein is a multiplicity of cell colonies comprising a library of colonies wherein each colony of the library contains an expression vector for the production of a different modular PKS, but derived from a naturally occurring PKS. The different PKS may be derived from the erythromycin PKS. In any case, the library of different modular PKS is obtained by modifying one or more of the regions of a naturally occurring gene or gene cluster encoding an enzymatic activity so as to alter that activity, leaving intact the scaffold portions of the naturally occurring gene. If desired, more than one scaffold source may be used, but basing the cluster of modules on a single scaffold is preferred. A multiplicity of cell colonies may comprise a library of colonies wherein each colony of the library contains a different modular PKS derived from a naturally occurring PKS, preferably the erythromycin PKS. Described herein are methods to produce libraries of PKS complexes and to produce libraries of polyketides by culturing these colonies, as well as to the libraries so produced. In addition, described herein are methods to screen the resulting polyketide libraries and to novel polyketides contained therein.

### Brief Description of the Drawings

Figure 1A is a diagram of the erythromycin PKS complex from *S*. *erythraeus* showing the function of each multifunctional protein, and also shows the structure of 6dEB and of D-desosamine and L-cladinose.
Figure 1B shows a diagram of the post-PKS biosynthesis of erythromycins A-D.
Figure 2 is a diagram of DEBS-1 from *S. erythraeus* showing the functional regions separated by linker regions.
Figure 3 shows a diagram of a vector containing the entire erythromycin gene cluster.
Figure 4 shows a method for the construction of the vector of Figure 3.
Figure 5 shows a diagram of the erythromycin gene cluster with locations of restriction sites introduced for ease of manipulation.
Figures 6A-6H show the structures of polyketides produced by manipulating the erythromycin PKS gene cluster.
Figures 7A (SEQ ID NO:1), 7B (SEQ ID NO:4) and 7C (SEQ ID NOS:1-3) show the construction of derivative PKS gene clusters from the vector of Figure 3.
Figure 8 shows antibiotics obtained from some of the polyketides of Figure 6A-6F.
Figure 9 shows a polyketide containing an unsaturated starter moiety and the corresponding antibiotic.
Figure 10 shows the preparation of a reagent used to glycosylate polyketides to prepare the D-desosamine derivatives with antibiotic activity.
Figure 11 shows the structures of known, previously produced, 12-member macrolides.
Figures 12A and 12B show the structures of known and previously produced 14-member macrolides.

### Modes of Canying Out the Invention

It may be helpful to review the nature of the erythromycin PKS complex and the gene cluster that encodes it as a model for modular PKS, in general.

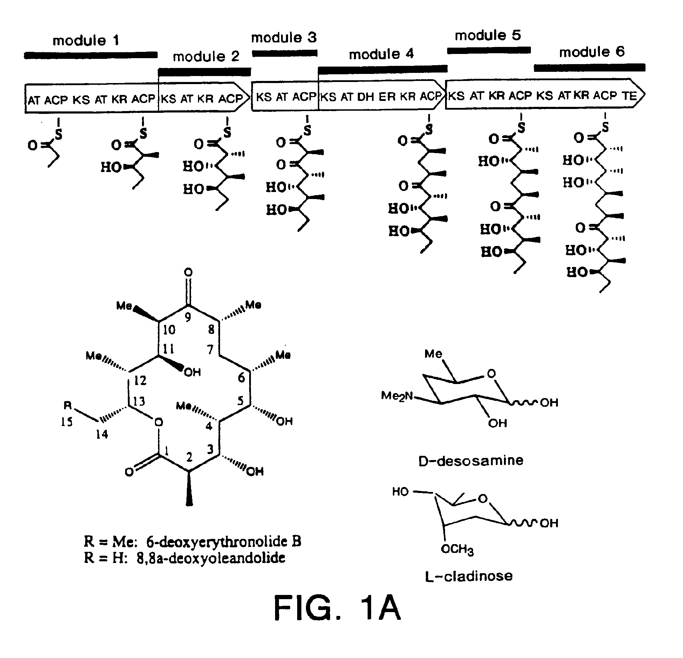

Figure 1A is a diagrammatic representation of the gene cluster encoding the synthase for the polyketide backbone of the antibiotic erythromycin. The erythromycin PKS protein assembly contains three high-molecular-weight proteins (>200 kD) designated DEBS-1, DEBS-2 and DEBS-3, each encoded by a separate gene (Caffrey et al., FEBS Lett (1992) 304:225). The diagram in Figure 1A shows that each of the three proteins contains two modules of the synthase -- a module being that subset of reactivities required to provide an additional 2-carbon unit to the molecule. As shown in Figure 1A, modules 1 and 2 reside on DEBS-1; modules 3 and 4 on DEBS-2 and modules 5 and 6 on DEBS-3. The minimal module is typified in module 3 which contains a ketosynthase (KS), an acyltransferase (AT) and an acyl carrier protein (ACP). These three functions are sufficient to activate an extender unit and attach it to the remainder of the growing molecule. Additional activities that may be included in a module relate to reactions other than the Claisen condensation, and include a dehydratase activity (DH), an enoylreductase activity (ER) and a ketoreductase activity (KR). The first module also contains repeats of the AT and ACP activities because it catalyzes the initial condensation, i.e. it begins with a "loading domain" represented by AT and ACP, which determine the nature of the starter unit. Although not shown, module 3 has a KR region which has been inactivated by mutation. The "finishing" of the molecule is regulated by the thioesterase activity (TE) in module 6. This thioesterase appears to catalyze cyclization of the macrolide ring thereby increasing the yield of the polyketide product.

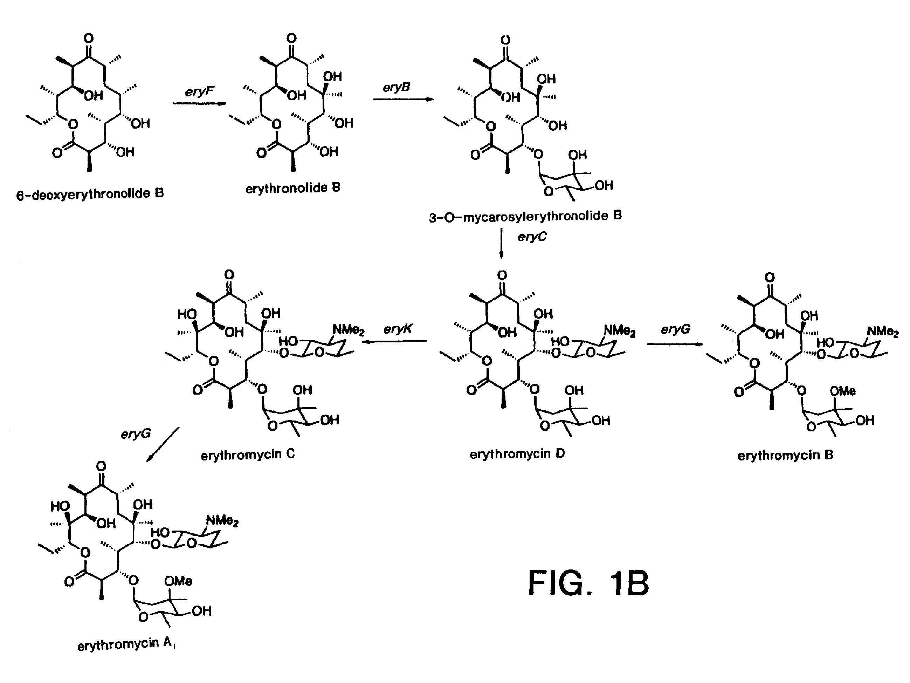

The product in this case is 6dEB; the structure and numbering system for this molecule are shown in Figure 1A. Conversion to the antibiotics erythromycin A, B, C and D would require glycosylation generally by D-desosamine or L-mycarose; the latter is converted to cladinose in erythromycins A and B. Figure 1B diagrams the post-PKS biosynthesis of the erythromycins, including addition and modification of glycosyl groups.

As shown, 6dEB is converted by the gene *eryF* to erythronolide B which is, in turn, glycosylated by *eryB* to obtain 3-O-mycarosylerythronolide B which contains L-mycarose at position 3. The enzyme *eryC* then converts this compound to erythromycin D by glycosylation with D-desosamine at position 5. Erythromycin D, therefore, differs from 6dEB through glycosylation and by the addition of a hydroxyl group at position 6. Erythromycin D can be converted to erythromycin B in a reaction catalyzed by *eryG* by methylating the L-mycarose residue at position 3. Erythromycin D is converted to erythromycin C by the addition of a hydroxyl group at position 12. Erythromycin A is obtained from erythromycin C by methylation of the mycarose residue catalyzed by *eryG.* The series of erythromycin antibiotics, then, differs by the level of hydroxylation of the polyketide framework and by the methylation status of the glycosyl residues.

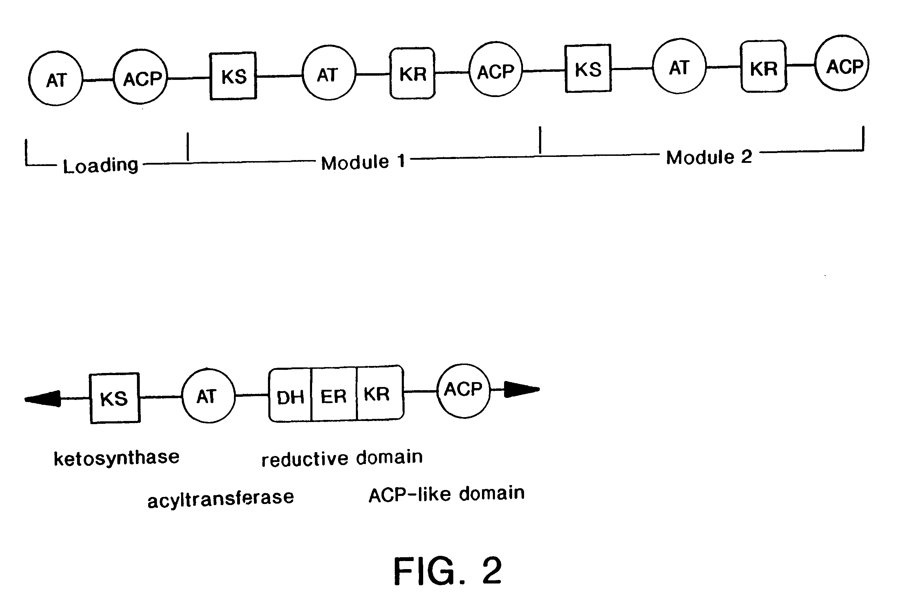

Figure 2 shows a detailed view of the regions in the first two modules which comprise the first open reading frame encoding DEBS-1. The regions that encode enzymatic activities are separated by linker or "scaffold"-encoding regions. These scaffold regions encode amino acid sequences that space the enzymatic activities at the appropriate distances and in the correct order. Thus, these linker regions collectively can be considered to encode a scaffold into which the various activities are placed in a particular order and spatial arrangement. This organization is similar in the remaining genes, as well as in other naturally occurring modular PKS gene clusters.

The three DEBS-1, 2 and 3 proteins are encoded by the genetic segments ery-AI, ery-AII and ery-AIII, respectively. These reading frames are located on the bacterial chromosome starting at about 10 kb distant from the erythromycin resistance gene (ermE or eryR).

The detailed description above referring to erythromycin is typical for modular PKS in general. Thus, rather than the illustrated erythromycin, the polyketide synthases making up the libraries described herein can be derived from the synthases of other modular PKS, such as those which result in the production of rapamycin, avermectin, FK-506, FR-008, monensin, rifamycin, soraphen-A, spinocyn, squalestatin, or tylosin, and the like.

Regardless of the naturally occurring PKS gene used as a scaffold, libraries or individual modified forms, ultimately of polyketides, may be produced by generating modifications in the erythromycin PKS or other naturally occurring PKS gene cluster so that the protein complexes produced by the cluster have altered activities in one or more respects, and thus produce polyketides other than the natural product of the PKS. Novel polyketides may thus be prepared, or polyketides in general prepared more readily, using this method. By providing a large number of different genes or gene clusters derived from a naturally occurring PKS gene cluster, each of which has been modified in a different way from the native cluster, an effectively combinatorial library of polyketides can be produced as a result of the multiple variations in these activities. All of the PKS encoding sequences used in the present invention represent modular polyketide synthases "derived from" a naturally occurring PKS, illustrated by the erythromycin PKS. As will be further described below, the metes and bounds of this derivation can be described on both the protein level and the encoding nucleotide sequence level.

By a modular PKS "derived from" the erythromycin or other naturally occurring PKS is meant a modular polyketide synthase (or its corresponding encoding gene(s)) that retains the scaffolding of all of the utilized portion of the naturally occurring gene. (Not all modules need be included in the constructs.) On the constant scaffold, at least one enzymatic activity is mutated, deleted or replaced, so as to alter the activity. Alteration results when these activities are deleted or are replaced by a different version of the activity, or simply mutated in such a way that a polyketide other than the natural product results from these collective activities. This occurs because there has been a resulting alteration of the starter unit and/or extender unit, and/or stereochemistry, and/or chain length or cyclization and/or reductive or dehydration cycle outcome at a corresponding position in the product polyketide. Where a deleted activity is replaced, the origin of the replacement activity may come from a corresponding activity in a different naturally occurring polyketide synthase or from a different region of the same PKS. In the case of erythromycin, for example, any or all of the DEBS-1, DEBS-2 and DEBS-3 proteins may be included in the derivative or portions of any of these may be included; but the scaffolding of an erythromycin PKS protein is retained in whatever derivative is considered. Similar comments pertain to the corresponding ery-AI, ery-AII and ery-AIII genes.

The derivative may contain preferably at least a thioesterase activity from the erythromycin or other naturally occurring PKS gene cluster.

In summary, a polyketide synthase "derived from" a naturally occurring PKS contains the scaffolding encoded by all or the portion employed of the naturally occurring synthase gene, contains at least two modules that are functional, preferably three modules, and more preferably four or more modules and contains mutations, deletions, or replacements of one or more of the activities of these functional modules so that the nature of the resulting polyketide is altered. This definition applies both at the protein and genetic levels. Particular preferred embodiments include those wherein a KS, AT, KR, DH or ER has been deleted or replaced by a version of the activity from a different PKS or from another location within the same PKS. Also preferred are derivatives where at least one noncondensation cycle enzymatic activity (KR, DH or ER) has been deleted or wherein any of these activities has been mutated so as to change the ultimate polyketide synthesized.

Thus, there are five degrees of freedom for constructing a polyketide synthase in terms of the polyketide that will be produced. First, the polyketide chain length will be determined by the number of modules in the PKS. Second, the nature of the carbon skeleton of the PKS will be determined by the specificities of the acyl transferases which determine the nature of the extender units at each position -- e.g.. malonyl, methyl malonyl, or ethyl malonyl, etc. Third, the loading domain specificity will also have an effect on the resulting carbon skeleton of the polyketide. Thus, the loading domain may use a different starter unit, such as acetyl, propionyl, and the like. Fourth, the oxidation state at various positions of the polyketide will be determined by the dehydratase and reductase portions of the modules. This will determine the presence and location of ketone, alcohol, double bonds or single bonds in the polyketide. Finally, the stereochemistry of the resulting polyketide is a function of three aspects of the synthase. The first aspect is related to the AT/KS specificity associated with substituted malonyls as extender units, which affects stereochemistry only when the reductive cycle is missing or when it contains only a ketoreductase since the dehydratase would abolish chirality. Second, the specificity of the ketoreductase will determine the chirality of any β-OH. Finally, the enoyl reductase specificity for substituted malonyls as extender units will influence the result when there is a complete KR/DH/ER available.

In the working examples below, the foregoing variables for varying loading domain specificity which controls the starter unit, a useful approach is to modify the KS activity in module 1 which results in the ability to incorporate alternative starter units as well as module 1 extended units. This approach was illustrated in PCT application WO97/02358 wherein the KS-I activity was inactivated through mutation. Polyketide synthesis is then initiated by feeding chemically synthesized analogs of module 1 diketide products. Working examples of this aspect are also presented hereinbelow.

Thus, the modular PKS systems, and in particular, the erythromycin PKS system, permit a wide range of polyketides to be synthesized. As compared to the aromatic PKS systems, a wider range of starter units including aliphatic monomers (acetyl, propionyl, butyryl, isovaleryl, etc.), aromatics (aminohydroxybenzoyl), alicyclics (cyclohexanoyl), and heterocyclics (thiazolyl) are found in various macrocyclic polyketides. Recent studies have shown that modular PKSs have relaxed specificity for their starter units (Kao *et al. Science* (1994), *supra*). Modular PKSs also exhibit considerable variety with regard to the choice of extender units in each condensation cycle. The degree of β-ketoreduction following a condensation reaction has also been shown to be altered by genetic manipulation (Donadio et al. Science (1991), *supra*; Donadio, S. et al. Proc Natl Acad Sci USA (1993) 90:7119-7123). Likewise, the size of the polyketide product can be varied by designing mutants with the appropriate number of modules (Kao, C. M. et al. JAm Chem Soc (1994) 116:11612-11613). Lastly, these enzymes are particularly well-known for generating an impressive range of asymmetric centers in their products in a highly controlled manner. The polyketides and antibiotics produced by the methods of the present invention are typically single stereoisomeric forms. Although the compounds can occur as mixtures of stereoisomers, it is more practical to generate individual stereoisomers using this system. Thus, the combinatorial potential within modular PKS pathways based on any naturally occurring modular, such as the erythromycin, PKS scaffold is virtually unlimited.

In general, the polyketide products of the PKS must be further modified, typically by glycosylation, in order to exhibit antibiotic activity. Methods for glycosylating the polyketides are generally known in the art; the glycosylation may be effected intracellularly by providing the appropriate glycosylation enzymes or may be effected *in vitro* using chemical synthetic means.

The antibiotic modular polyketides may contain any of a number of different sugars, although D-desosamine, or a close analog thereof, is most common. Erythromycin, picromycin, narbomycin and methymycin contain desosamine. Erythromycin also contains L-cladinose (3-O-methyl mycarose). Tylosin contains mycaminose (4-hydroxy desosamine), mycarose and 6-deoxy-D-allose. 2-acetyl-1-bromodesosamine has been used as a donor to glycosylate polyketides by Masamune et al. JAm Chem Soc (1975) 97:3512, 3513. Other, apparently more stable, donors include glycosyl fluorides, thioglycosides, and trichloroacetimidates; Woodward, R.B. et al. J Am Chem Soc (1981) 103:3215; Martin, S.F. et al. Am Chem Soc (1997) 119:3193; Toshima, K. et al. JAm Chem Soc (1995) 117:3717; Matsumoto, T. et al. Tetrahedron Lett (1988) 29:3575. Glycosylation can also be effected using the macrolides as starting materials and using mutants of *S*. *erythraea* that are unable to synthesize the macrolides to make the conversion. A method is illustrated in the Examples hereinbelow.

### Methods to Construct Multiple Modular PKS Derived from a Naturally Occurring PKS

The derivatives of a naturally occurring PKS can be prepared by manipulation of the relevant genes. A large number of modular PKS gene clusters have been mapped and/or sequenced, including erythromycin, soraphen A, rifamycin, and rapamycin, which have been completely mapped and sequenced, and FK506 and oleandomycin which have been partially sequenced, and candicidin, avermectin, and nemadectin which have been mapped and partially sequenced. Additional modular PKS gene clusters are expected to be available as time progresses. These genes can be manipulated using standard techniques to delete or inactivate activity encoding regions, insert regions of genes encoding corresponding activities from the same or different PKS system, or otherwise mutated using standard procedures for obtaining genetic alterations. Of course, portions of, or all of, the desired derivative coding sequences can be synthesized using standard solid phase synthesis methods such as those described by Jaye et al., J Biol Chem (1984) 259:6331 and which are available commercially from, for example, Applied Biosystems, Inc.

In order to obtain nucleotide sequences encoding a variety of derivatives of the naturally occurring PKS. and thus a variety of polyketides for construction of a library, a desired number of constructs can be obtained by "mixing and matching" enzymatic activity-encoding portions, and mutations can be introduced into the native host PKS gene cluster or portions thereof.

Mutations can be made to the native sequences using conventional techniques. The substrates for mutation can be an entire cluster of genes or only one or two of them; the substrate for mutation may also be portions of one or more of these genes. Techniques for mutation include preparing synthetic oligonucleotides including the mutations and inserting the mutated sequence into the gene encoding a PKS subunit using restriction endonuclease digestion. (See, *e.g*., Kunkel, T.A. Proc Natl Acad Sci USA (1985) 82:448; Geisselsoder et al. BioTechniques (1987) 5:786.) Alternatively, the mutations can be effected using a mismatched primer (generally 10-20 nucleotides in length) which hybridizes to the native nucleotide sequence (generally cDNA corresponding to the RNA sequence), at a temperature below the melting temperature of the mismatched duplex. The primer can be made specific by keeping primer length and base composition within relatively narrow limits and by keeping the mutant base centrally located. Zoller and Smith, Methods Enzymol (1983) 100:468. Primer extension is effected using DNA polymerase, the product cloned and clones containing the mutated DNA, derived by segregation of the primer extended strand, selected. Selection can be accomplished using the mutant primer as a hybridization probe. The technique is also applicable for generating multiple point mutations. *See, e.g*., Dalbie-McFarland et al. Proc Natl Acad Sci USA (1982) 79:6409. PCR mutagenesis will also find use for effecting the desired mutations.

Random mutagenesis of selected portions of the nucleotide sequences encoding enzymatic activities can be accomplished by several different techniques known in the art, e.g., by inserting an oligonucleotide linker randomly into a plasmid, by irradiation with X-rays or ultraviolet light, by incorporating incorrect nucleotides during *in vitro* DNA synthesis, by error-prone PCR mutagenesis, by preparing synthetic mutants or by damaging plasmid DNA *in vitro* with chemicals. Chemical mutagens include, for example, sodium bisulfite, nitrous acid, nitrosoguanidine, hydroxylamine, agents which damage or remove bases thereby preventing normal base-pairing such as hydrazine or formic acid, analogues of nucleotide precursors such as 5-bromouracil, 2-aminopurine, or acridine intercalating agents such as proflavine, acriflavine, quinacrine, and the like. Generally, plasmid DNA or DNA fragments are treated with chemicals, transformed into *E. coli* and propagated as a pool or library of mutant plasmids.

In addition to providing mutated forms of regions encoding enzymatic activity, regions encoding corresponding activities from different PKS synthases or from different locations in the same PKS synthase can be recovered, for example, using PCR techniques with appropriate primers. By "corresponding" activity encoding regions is meant those regions encoding the same general type of activity -- e.g., a ketoreductase activity in one location of a gene cluster would "correspond" to a ketoreductase-encoding activity in another location in the gene cluster or in a different gene cluster; similarly, a complete reductase cycle could be considered corresponding -- e.g., KR/DH/ER would correspond to KR alone.

If replacement of a particular target region in a host polyketide synthase is to be made, this replacement can be conducted *in vitro* using suitable restriction enzymes or can be effected *in vivo* using recombinant techniques involving homologous sequences framing the replacement gene in a donor plasmid and a receptor region in a recipient plasmid. Such systems, advantageously involving plasmids of differing temperature sensitivities are described, for example, in PCT publication WO 96/40968.

The vectors used to perform the various operations to replace the enzymatic activity in the host PKS genes or to support mutations in these regions of the host PKS genes may be chosen to contain control sequences operably linked to the resulting coding sequences in a manner that expression of the coding sequences may be effected in a appropriate host. However, simple cloning vectors may be used as well.

If the cloning vectors employed to obtain PKS genes encoding derived PKS lack control sequences for expression operably linked to the encoding nucleotide sequences, the nucleotide sequences are inserted into appropriate expression vectors. This need not be done individually, but a pool of isolated encoding nucleotide sequences can be inserted into host vectors, the resulting vectors transformed or transfected into host cells and the resulting cells plated out into individual colonies.

Suitable control sequences include those which function in eucaryotic and procaryotic host cells. Preferred hosts include fungal systems such as yeast and procaryotic hosts, but single cell cultures of, for example, mammalian cells could also be used. There is no particular advantage, however, in using such systems. Particularly preferred are yeast and procaryotic hosts which use control sequences compatible with *Streptomyces spp.* Suitable controls sequences for single cell cultures of various types of organisms are well known in the art. Control systems for expression in yeast, including controls which effect secretion are widely available and routinely used. Control elements include promoters, optionally containing operator sequences, and other elements depending on the nature of the host, such as ribosome binding sites. Particularly useful promoters for procaryotic hosts include those from PKS gene clusters which result in the production of polyketides as secondary metabolites, including those from aromatic (Type II) PKS gene clusters. Examples are act promoters, tcm promoters, spiramycin promoters, and the like. However. other bacterial promoters, such as those derived from genes that encode sugar metabolizing enzymes, such as galactose, lactose (*lac*) and maltose, are also useful. Additional examples include promoters derived from genes that encode biosynthetic enzymes for compounds such as tryptophan (*trp*), and the β-lactamase (*bla*), bacteriophage lambda PL, and T5 promoters. In addition, synthetic promoters, such as the *tac* promoter (U.S. Patent No. 4,551,433), can be used.

Other regulatory sequences may also be desirable which allow for regulation of expression of the PKS replacement sequences relative to the growth of the host cell. Regulatory sequences are known to those of skill in the art, and examples include those which cause the expression of a gene to be turned on or off in response to a chemical or physical stimulus, including the presence of a regulatory compound. Other types of regulatory elements may also be present in the vector, for example, enhancer sequences.

Selectable markers can also be included in the recombinant expression vectors. A variety of markers are known which are useful in selecting for transformed cell lines and generally comprise a gene whose expression confers a selectable phenotype on transformed cells when the cells are grown in an appropriate selective medium. Such markers include, for example, genes which confer antibiotic resistance or sensitivity to the plasmid. Alternatively, several polyketides are naturally colored and this characteristic provides a built-in marker for screening cells successfully transformed by the present constructs.

The various PKS nucleotide sequences, or a cocktail of such sequences, can be cloned into one or more recombinant vectors as individual cassettes, with separate control elements, or under the control of, e.g., a single promoter. The PKS subunits or cocktail components can include flanking restriction sites to allow for the easy deletion and insertion of other PKS subunits or cocktail components so that hybrid PKSs can be generated. The design of such unique restriction sites is known to those of skill in the art and can be accomplished using the techniques described above, such as site-directed mutagenesis and PCR.

As described above, particularly useful control sequences are those which themselves, or using suitable regulatory systems, activate expression during transition from growth to stationary phase in the vegetative mycelium. The system contained in the illustrated plasmid pCK7, i.e., the *act*I/*act*III promoter pair and the *act*II-ORF4, an activator gene, is particularly preferred. Particularly preferred hosts are those which lack their own means for producing polyketides so that a cleaner result is obtained. Illustrative host cells of this type include the modified *S*. *coelicolor* CH999 culture described in PCT application WO 96/40968 and similar strains of *S*. *lividans.*

The expression vectors containing nucleotide sequences encoding a variety of PKS systems for the production of different polyketides are then transformed into the appropriate host cells to construct the library. In one straightforward approach, a mixture of such vectors is transformed into the selected host cells and the resulting cells plated into individual colonies and selected for successfui transformants. Each individual colony will then represent a colony with the ability to produce a particular PKS synthase and ultimately a particular polyketide. Typically, there will be duplications in some of the colonies; the subset of the transformed colonies that contains a different PKS in each member colony can be considered the library. Alternatively, the expression vectors can be used individually to transform hosts, which transformed hosts are then assembled into a library. A variety of strategies might be devised to obtain a multiplicity of colonies each containing a PKS gene cluster derived from the naturally occurring host gene cluster so that each colony in the library produces a different PKS and ultimately a different polyketide. The number of different polyketides that are produced by the library is typically at least four, more typically at least ten, and preferably at least 20, more preferably at least 50, reflecting similar numbers of different altered PKS gene clusters and PKS gene products. The number of members in the library is arbitrarily chosen; however, the degrees of freedom outlined above with respect to the variation of starter, extender units, stereochemistry, oxidation state, and chain length is quite large.

Methods for introducing the recombinant vectors of the present invention into suitable hosts are known to those of skill in the art and typically include the use of CaCl₂ or other agents, such as divalent cations, lipofection, DMSO, protoplast transformation and electroporation.

As disclosed in copending application Serial No. 08/989,332 filed 11 December 1997, (US Patent No. 6,033,883) a wide variety of hosts can be used, even though some hosts natively do not contain the appropriate post-translational mechanisms to activate the acyl carrier proteins of the synthases. These hosts can be modified with the appropriate recombinant enzymes to effect these modifications.

The polyketide producing colonies can be identified and isolated using known techniques and the produced polyketides further characterized. The polyketides produced by these colonies can be used collectively in a panel to represent a library or may be assessed individually for activity.

The libraries can thus be considered at four levels: (1) a multiplicity of colonies each with a different PKS encoding sequence encoding a different PKS cluster but all derived from a naturally occurring PKS cluster; (2) colonies which contain the proteins that are members of the PKS produced by the coding sequences; (3) the polyketides produced; and (4) antibiotics derived from the polyketides. Of course, combination libraries can also be constructed wherein members of a library derived, for example, from the erythromycin PKS can be considered as a part of the same library as those derived from, for example, the rapamycin PKS cluster.

Colonies in the library are induced to produce the relevant synthases and thus to produce the relevant polyketides to obtain a library of candidate polyketides. The polyketides secreted into the media can be screened for binding to desired targets, such as receptors, signaling proteins, and the like. The supernatants *per se* can be used for screening, or partial or complete purification of the polyketides can first be effected. Typically, such screening methods involve detecting the binding of each member of the library to receptor or other target ligand. Binding can be detected either directly or through a competition assay. Means to screen such libraries for binding are well known in the art.

Alternatively, individual polyketide members of the library can be tested against a desired target. In this event, screens wherein the biological response of the target is measured can more readily be included.

Indeed, a large number of novel polyketides have been prepared according to the method as illustrated in the examples below. These novel polyketides are useful intermediates in formation of compounds with antibiotic activity through glycosylation reactions as described above. As indicated above, the individual polyketides are reacted with suitable sugar derivatives to obtain compounds of antibiotic activity. Antibiotic activity can be verified using typical screening assays such as those set forth in Lehrer, R. et al. J Immunol Meth (1991) 137:167-173.

New polyketides are those described below. New antibiotics include the glycosylated forms of these polyketides.

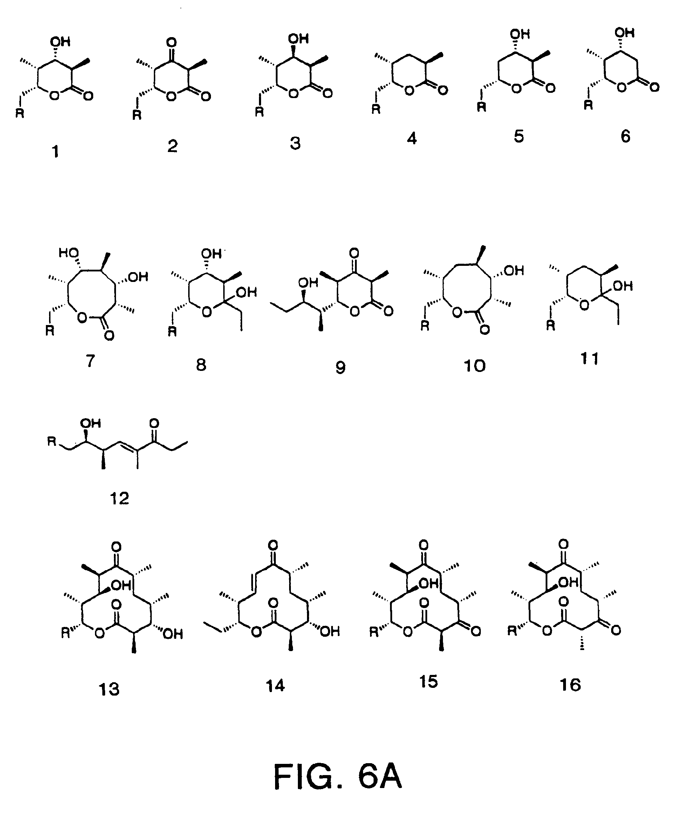

The polyketides include the compounds of structure (1) and the glycosylated forms thereof. Thecompounds include the polyketide structure: 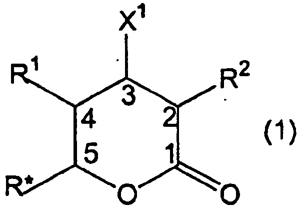 including the isolated stereoisomeric forms thereof;
wherein R* is a straight chain, branched or cyclic, saturated or unsaturated substituted or unsubstituted hydrocarbyl of 1-15C;
each of R¹ and R² is independently H or alkyl (1-4C) wherein any alkyl at R¹ may optionally be substituted;
X¹ is H₂, HOH or =O;
with the provisos that:
   at least one of R¹ and R² must be alkyl (1-4C); and
   the compound is other than compounds 1, 2, 3, 5 and 6 of Figure 6A.

Particularly preferred embodiments of formula (1) include compound 4 shown in Figure 6A.

The polyketides include the compounds of formula (2) and the glycosylated forms thereof. These compounds include the polyketide structure: 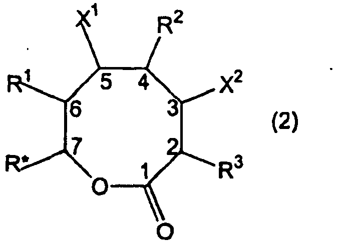 including the isolated stereoisomeric forms thereof;
wherein R* is a straight chain, branched or cyclic, saturated or unsaturated substituted or unsubstituted hydrocarbyl of 1-15C;
each of R¹, R² and R³ is independently H or alkyl (1-4C) wherein any alkyl at R¹ may optionally be substituted;
each of X¹ and X² is independently H₂, HOH or =O;
with the provisos that:
   at least two of R¹, R² and R³ are alkyl (1-4C).

The polyketides include the compounds of structure (3) and the glycosylated forms thereof. Thecompounds include the polyketide structure: 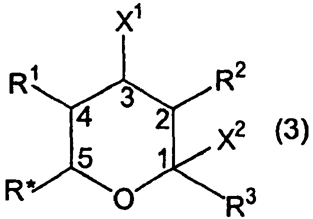 including the isolated stereoisomeric forms thereof;
wherein R* is a straight chain, branched or cyclic, saturated or unsaturated substituted or unsubstituted hydrocarbyl of 1-15C;
each of R¹, R² and R³ is independently H or alkyl (1-4C) wherein any alkyl at R¹ may optionally be substituted;
each of X¹ and X² is independently H₂, HOH or =O;
with the provisos that:
   at least one of R¹ and R² must be alkyl (1-4C); and
   the compound is other than compound 8 of Figure 6A.

The antibiotic forms of the polyketide of formula (3) are the corresponding glycosylated forms.

Still other compounds are those of the following formula, including the glycosylated forms thereof. These are derived from the compound of formula (4) which has the structure: 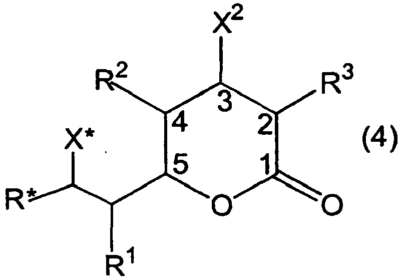 including the isolated stereoisomeric forms thereof;
wherein R* is a straight chain, branched or cyclic, saturated or unsaturated substituted or unsubstituted hydrocarbyl of 1-15C;
each of R¹, R² and R³ is independently H or alkyl (1-4C) wherein any alkyl at R¹ may optionally be substituted;
each of X* and X² is independently H₂, HOH or =O;
with the proviso that:
   at least one of R² and R³ is alkyl (1-4C); and
   the compound is other than compound 9 of Figure 6A.

Still other compounds are the result of the condensation of five modules of the polyketide synthase system. The polyketide forms of these compounds are of the formula: 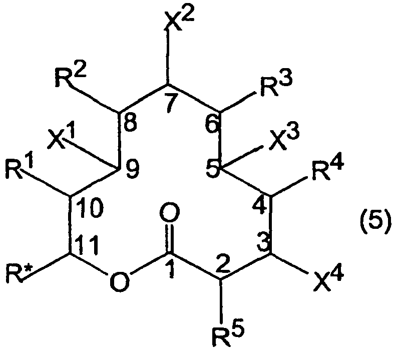 including the glycosylated and isolated stereoisomeric forms thereof;
wherein R* is a straight chain, branched or cyclic, saturated or unsaturated substituted or unsubstituted hydrocarbyl of 1-15C;
each of R¹, R², R³, R⁴ and R⁵ is independently H or alkyl (1-4C) wherein any alkyl at R¹ may optionally be substituted;
each of X¹, X², X³ and X⁴ is independently H₂, HOH or =O; or
X¹ or X² or X³ or X⁴ is H and the compound of formula (5) contains a π-bond at positions 8-9 or 6-7 or 4-5 or 2-3;
with the proviso that:
   at least two of R¹-R⁵ are alkyl (1-4C); and
   the compound is other than compound 13 or 14 of Figure 6A or compound 205, 210-213 of figure 11.

Preferred forms of compounds that include formula (5) as those wherein at least three, more preferably at least four, of R¹-R⁵ are alkyl (1-4C), preferably methyl or ethyl.

Also preferred are compounds wherein X¹ is -OH and/or X² =O, and/or X³ is H.

The glycosylated forms of these compounds are also useful antibiotics.

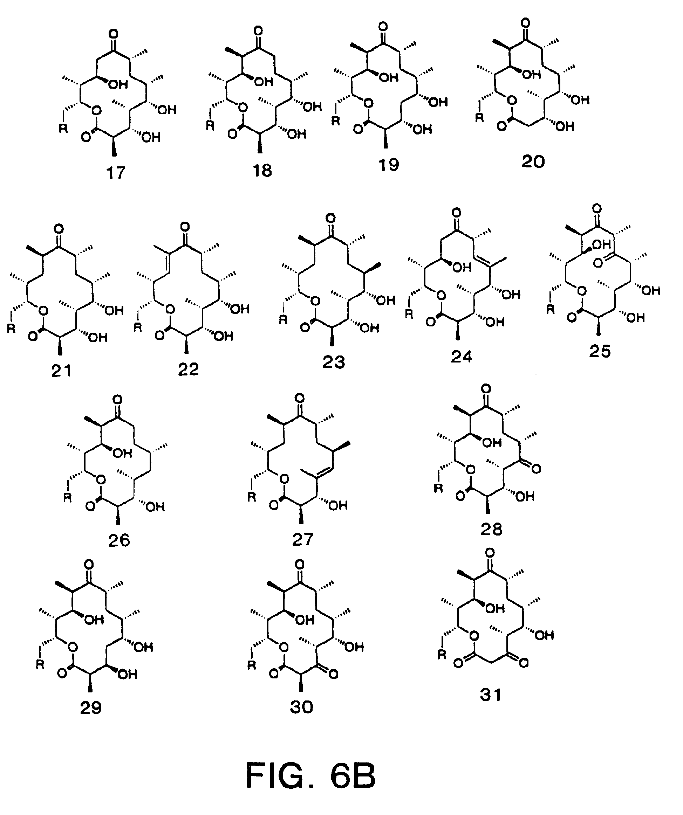

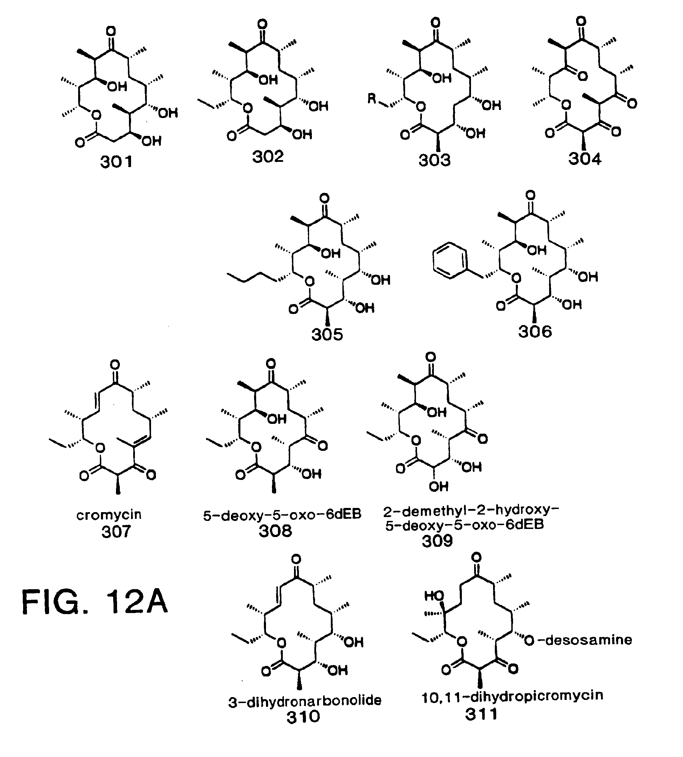

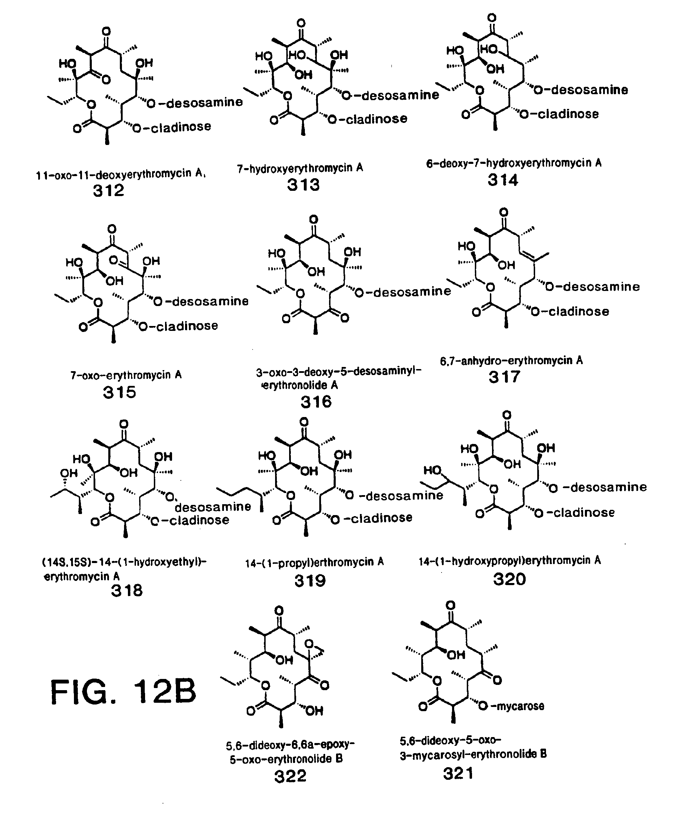

Resulting from the condensation effected by six modules are the compounds which comprise the formula: 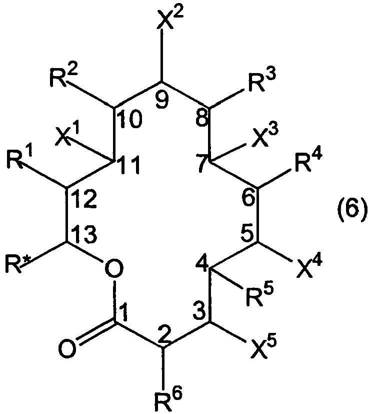 including the glycosylated and isolated stereoisomeric forms thereof;
wherein R* is a straight chain, branched or cyclic, saturated or unsaturated substituted or unsubstituted hydrocarbyl of 1-15C;
each of R¹-R⁶ is independently H or alkyl (1-4C) wherein any alkyl at R¹ may optionally be substituted;
each of X¹-X⁵ is independently H₂, HOH or =O; or
each of X¹-X⁵ is independently H and the compound of formula (5) contains a π-bond in the ring adjacent to the position of said X at 2-3, 4-5, 6-7, 8-9 and/or 10-11;
with the proviso that:
   at least two of R¹-R⁶ are alkyl (1-4C); and
   the compound is other than compound 17, 24 or 28 of Figure 6B, compound 301-311 of Figure 12(A) or compounds 312-322 of Figure 12(B).

Preferred compounds comprising formula 6 are those wherein at least three of R¹-R⁵ are alkyl (1-4C), preferably methyl or ethyl; more preferably wherein at least four of R¹-R⁵ are alkyl (1-4C), preferably methyl or ethyl.

Also preferred are those wherein X² is H₂, =O or H►···OH, and/orX³ is H, and/or X¹ is OH and/or X⁴ is OH and/or X⁵ is OH.

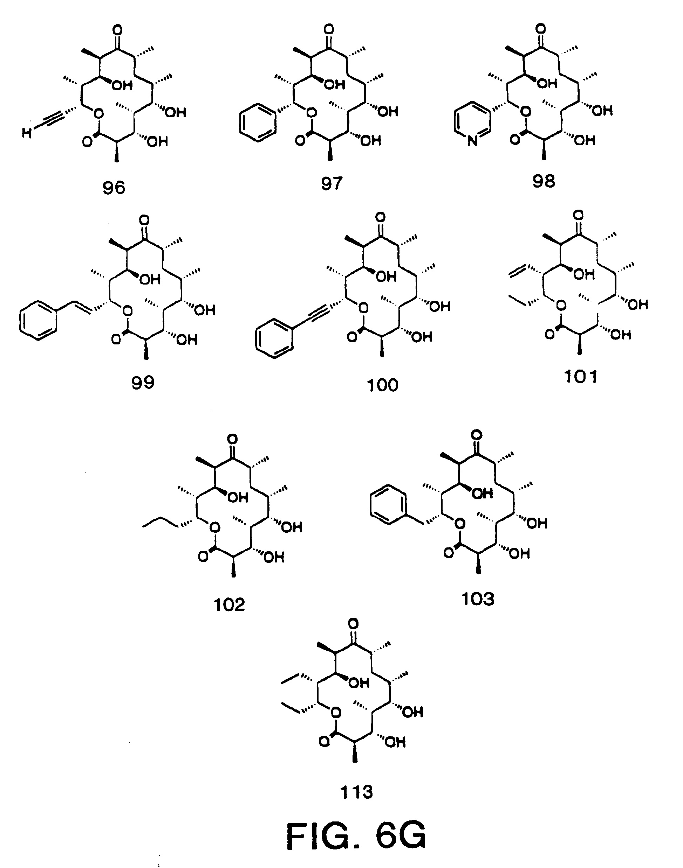

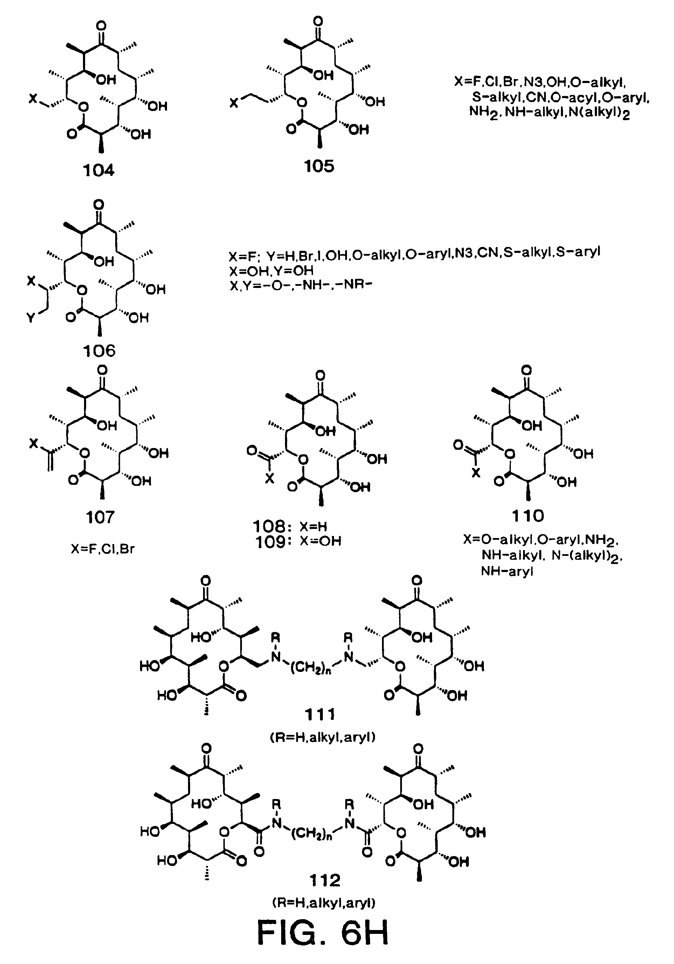

Particularly preferred are compounds of formulas 18-23, 25-27, 29-75 and 101 and 113 of Figures 6B-6F. Also preferred are compounds with variable R* when R¹-R⁵ are methyl, X² is =O, and X¹, X⁴ and X⁵ are OH examples of which are depicted in formulas 96-100 and 104-107 of Figures 6G and 6H. The glycosylated forms of the foreoging are also preferred.

Other polyketides which result from the condensation catalyzed by six modules of a modular PKS include those of the formula: 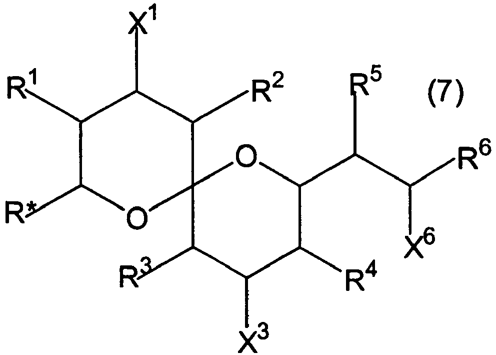 including the glycosylated and isolated stereoisomeric forms thereof;
wherein R* is a straight chain, branched or cyclic, saturated or unsaturated substituted or unsubstituted hydrocarbyl of 1-15C;
each of R¹-R⁵ is independently H or alkyl (1-4C) wherein any alkyl at R¹ may optionally be substituted;
R⁶ is alkyl (1-5C);
each of X¹ and X³ and X⁶ is independently H₂, HOH or =O;
with the proviso that:
   at least two of R¹-R⁴ are alkyl (1-4C).

Still others include those of the formula: 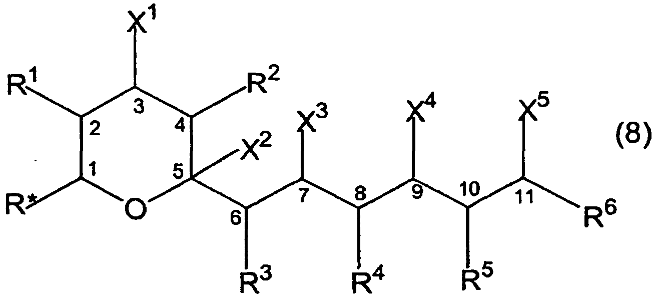 including the isolated stereoisomeric forms thereof;
wherein R* is a straight chain, branched or cyclic, saturated or unsaturated substituted or unsubstituted hydrocarbyl of 1-15C;
each of R¹-R⁵ is independently H or alkyl (1-4C) wherein any alkyl at R¹ may optionally be substituted;
R⁶ is alkyl (1-5C);
X² is OH or H;
each X¹, X³, X⁴ and X⁵ is independently H₂, HOH or =O; or X³ or X⁴ is H and
the compound of formula (8) has a π-bond between positions 7-8 or 9-10, with the proviso that:
   if X² is H, at least one of X³ and X⁴ is HOH or =O.

As above, the glycosylated forms are useful antibiotics.

As set forth above, R* in the compounds described herein may be substituted as well as unsubstituted. Suitable substituents include halo (F, Cl, Br, I), N₃, OH, O-alkyl (1-6C), S-alkyl (1-6C), CN, O-acyl (1-7C), O-aryl (6-10C), O-alkyl-aryl (7-14C), NH₂, NH-alkyl (1-6C) and N-(alkyl)₂.

Suitable substituents on R¹ are selected from the same group as those for R* In addition, the substituents on R¹ and R* may form a ring system such as an epoxide ring, or a larger heterocyclic ring including O, or N or S. Preferred substituents for R* and R¹ are halo, OH and NH₂. Unsubstituted forms are also preferred.

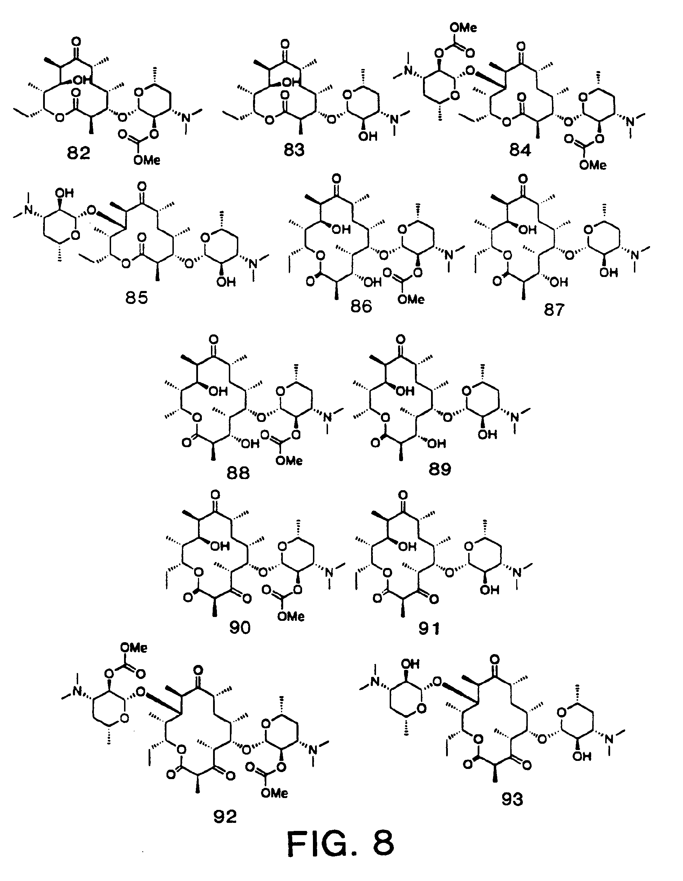

Particularly useful as antibiotics are compounds of formulas 82-93 as set forth in Figure 8 herein.

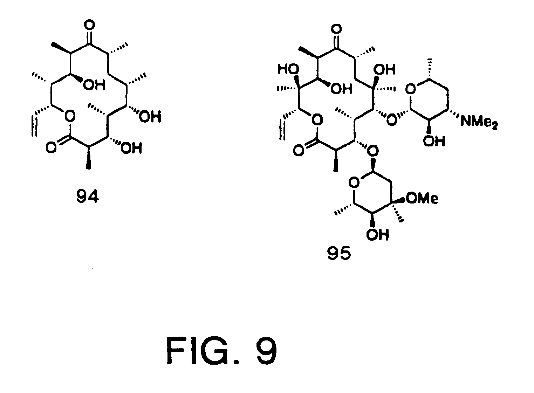

Still another compound is set forth as compound 94 in Figure 9. Its glycosylated form, shown as compound 95, is useful as an antibiotic.

### Examples

The following examples are intended to illustrate, but not to limit the invention.

### Materials and Methods

### General Techniques:

**Bacterial strains, plasmids, and culture conditions.** *S*. *coelicolor* CH999 described in WO 95/08548, published 30 March 1995 was used as an expression host. DNA manipulations were performed in *Escherichia coli* MC1061. Plasmids were passaged through *E*. *coli* ET12567 (*dam dcm hsdS* Cm^{r}) (MacNeil, D.J. J Bacteriol (1988) 170:5607) to generate unmethylated DNA prior to transformation of *S. coelicolor. E. coli* strains were grown under standard conditions. *S*. *coelicolor* strains were grown on R2YE agar plates (Hopwood, D.A. et al. Genetic manipulation of Streptomyces. A laboratory manual. The John Innes Foundation: Norwich, 1985). pRM5, also described in WO 95/08548, includes a colEI replicon, an appropriately truncated SCP2* *Streptomyces* replicon, two *act*-promoters to allow for bidirectional cloning, the gene encoding the actII-ORF4 activator which induces transcription from *act* promoters during the transition from growth phase to stationary phase, and appropriate marker genes. Engineered restriction sites facilitate the combinatorial construction of PKS gene clusters starting from cassettes encoding individual domains of naturally occurring PKSs.

When pRM5 is used for expression of PKS, (i) all relevant biosynthetic genes are plasmid-borne and therefore amenable to facile manipulation and mutagenesis in *E. coli,* (ii) the entire library of PKS gene clusters can be expressed in the same bacterial host which is genetically and physiologically well-characterized and presumably contains most, if not all, ancillary activities required for *in vivo* production of polyketides, (iii) polyketides are produced in a secondary metabolite-like manner, thereby alleviating the toxic effects of synthesizing potentially bioactive compounds *in vivo,* and (iv) molecules thus produced undergo fewer side reactions than if the same pathways were expressed in wild-type organisms or blocked mutants.

Manipulation of DNA and organisms. Polymerase chain reaction (PCR) was performed using Taq polymerase (Perkin Elmer Cetus) under conditions recommended by the enzyme manufacturer. Standard *in vitro* techniques were used for DNA manipulations (Sambrook, et al. Molecular Cloning: A Laboratory Manual (Current Edition)). *E. coli* was transformed with a Bio-Rad *E*. *Coli* Pulsing apparatus using protocols provided by Bio-Rad. S. *coelicolor* was transformed by standard procedures (Hopwood, D.A. *et al. Genetic manipulation of Streptomyces. A laboratory manual.* The John Innes Foundation: Norwich, 1985) and transformants were selected using 2 mL of a 500 µg/ml thiostrepton overlay.

### Preparation A

### Construction of the Complete Erythromycin PKS Gene Cluster

### Recovery of the Erythromycin PKS Genes

Although various portions of the erythromycin PKS gene cluster can be manipulated separately at any stage of the process of preparing libraries, it may be desirable to have a convenient source of the entire gene cluster in one place. Thus, the entire erythromycin PKS gene cluster can be recovered on a single plasmid if desired. This is illustrated below utilizing derivatives of the plasmid pMAK705 (Hamilton *et al. J Bacteriol* (1989) 171:4617) to permit *in vivo* recombination between a temperature-sensitive donor plasmid, which is capable of replication at a first, permissive temperature and incapable of replication at a second, non-permissive temperature, and recipient plasmid. The *eryA* genes thus cloned gave pCK7, a derivative of pRM5 (McDaniel et al. Science (1993) 262:1546). A control plasmid. pCK7f, was constructed to carry a frameshift mutation in *eryA1.* pCK7 and pCK7f possess a *ColE*I replicon for genetic manipulation in *E. coli* as well as a truncated SCP2* (low copy number) *Streptomyces* replicon.

These plasmids also contain the divergent *act*I/*act*III promoter pair and *act*II-ORF4, an activator gene, which is required for transcription from these promoters and activates expression during the transition from growth to stationary phase in the vegetative mycelium. High-level expression of PKS genes occurs at the onset of the stationary phase of mycelial growth. The recombinant strains therefore produce the encoded polyketides as secondary metabolites.

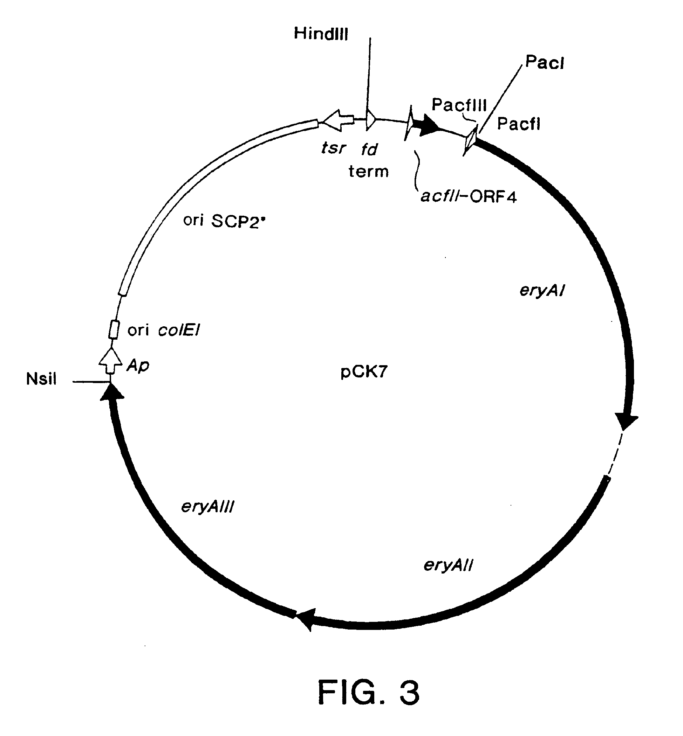

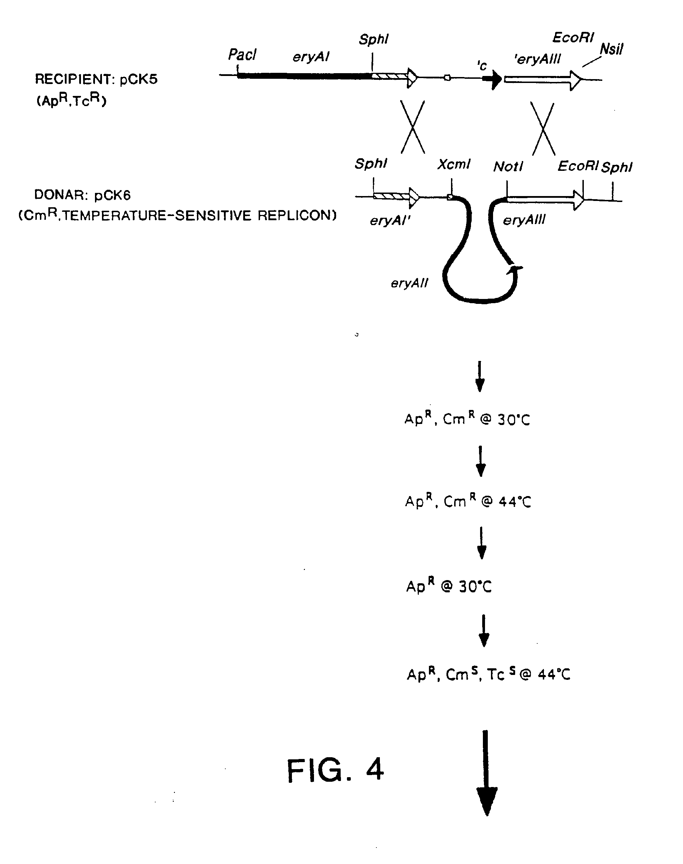

In more detail, pCK7 (Figure 3), a shuttle plasmid containing the complete *eryA* genes, which were originally cloned from pS1 (Tuan et al. Gene (1990) 90:21), was constructed as follows. The modular DEBS PKS genes were transferred incrementally from a temperature-sensitive "donor" plasmid, i.e., a plasmid capable of replication at a first, permissive temperature and incapable of replication at a second, non-permissive temperature, to a "recipient" shuttle vector via a double recombination event, as depicted in Figure 4. A 25.6 kb *Sph*I fragment from pS1 was inserted into the *Sph*I site of pMAK705 (Hamilton et al. J Bacteriol (1989) 171:4617) to give pCK6 (Cm^{R}), a donor plasmid containing *eryAII. eryAIII*, and the 3' end of *eryAI.* Replication of this temperature-sensitive pSC101 derivative occurs at 30°C but is arrested at 44°C. The recipient plasmid, pCK5 (Ap^{R}, Tc^{R}), includes a 12.2 kb *eryA* fragment from the *eryAI* start codon (Caffrey et al. FEBS Lett (1992) 304:225) to the *Xcm*I site near the beginning of *eryAII,* a 1.4 kb *EcoR*I*-Bsm*I pBR322 fragment encoding the tetracycline resistance gene (*Tc*), and a 4.0 kb *Not*I*-EcoR*I fragment from the end of *eryAIII. Pac*I, *Nde*I, and ribosome binding sites were engineered at the *eryAI* start codon in pCK55. pCK5 is a derivative of pRM5 (described above). The 5' and 3' regions of homology are 4.1 kb and 4.0 kb, respectively. MC1061 *E. coli* was transformed with pCK5 and pCK6 and subjected to carbenicillin and chloramphenicol selection at 30°C. Colonies harboring both plasmids (Ap^{R}, Cm^{R}) were then restreaked at 44°C on carbenicillin and chloramphenicol plates. Only cointegrates formed by a single recombination event between the two plasmids were viable. Surviving colonies were propagated at 30°C under carbenicillin selection, forcing the resolution of the cointegrates via a second recombination event. To enrich for pCK7 recombinants, colonies were restreaked again on carbenicillin plates at 44°C. Approximately 20% of the resulting colonies displayed the desired phenotype (Ap^{R}, Tc^{S}, Cm^{S}). The final pCK7 candidates were thoroughly checked via restriction mapping. A control plasmid, pCK7f, which contains a frameshift error in *eryAI,* was constructed in a similar manner. pCK7 and pCK7f were transformed into *E. coli* ET12567 (MacNeil J Bacteriol (1988) 170:5607) to generate unmethylated plasmid DNA and subsequently moved into *Streptomyces coelicolor* CH999.

Upon growth of CH999/pCK7 on R2YE medium, the organism produced abundant quantities of two polyketides. The addition of propionate (300 mg/L) to the growth medium resulted in approximately a two-fold increase in yield of polyketide product. Proton and ¹³C NMR spectroscopy, in conjunction with propionic-1-¹³C acid feeding experiments, confirmed the major product as 6dEB (>40 mg/L) (Figure 1A). The minor product was identified as 8,8a-deoxyoleandolide (>10 mg/L) (Figure 1A), which apparently originates from an acetate starter unit instead of propionate in the 6dEB biosynthetic pathway. ¹³C₂ sodium acetate feeding experiments confirmed the incorporation of acetate into the minor product. Three high molecular weight proteins (>200 kDa), presumably DEBS1, DEBS2, and DEBS3 (Caffrey et al. FEBS Lett (1992) 304:225), were also observed in crude extracts of CH999/pCK7 via SDS-polyacrylamide gel electrophoresis. No polyketide products were observed from CH999/pCK7f. The inventors hereby acknowledge support provided by the American Cancer Society (IRG-32-34).

### Example 1

### Preparation of Scaffolds for Replacing DEBS AT and KR Domains

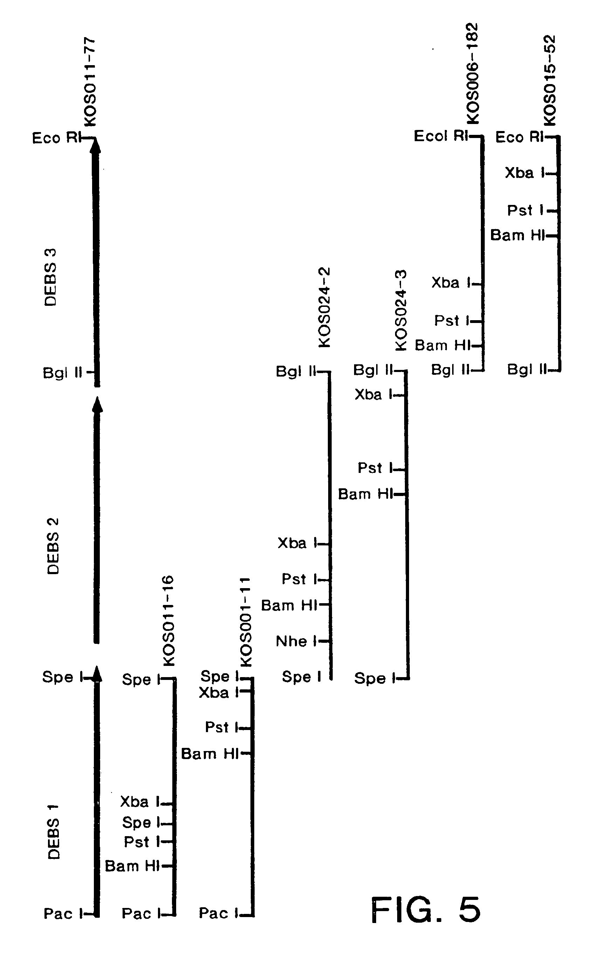

For each of the six modules of DEBS, a subclone was made containing endonuclease restriction sites engineered at selected boundaries of the acyltransferase (AT) and reduction (KR or DH/ER/KR) domains. The restriction sites were introduced into the subclones by PCR mutagenesis. A *Bam*HI site was used for the 5' boundary of AT domains, a *Pst*I site was introduced between the AT and reductive domains, and *Xba*I was used ai the 3' end of the reductive domain (see Figure 5). This resulted in the following engineered sequences (lowercase indicates engineered restriction site) (SEQ ID NOS:5-22):

| Module 1 (pKOS011-16) | |
|---|---|
| 5' AT boundary | GCGCAGCAG*ggatcc*GTCTTCGTC |
| AT/KR boundary | CGCGTCTGG*ctgcag*CCGAAGCCG |
| 3' KR boundary | CCGGCCGAAt*ctaga*GTGGGCGCG |
| | |

| Module 2 (pKOS001-11) | |
|---|---|
| 5' AT boundary | TCCGACGGT*ggatcc*GTGTTCGTC |
| AT/KR boundary | CGGTTCTGG*ctgcag*CCGGACCGC |
| 3' KR boundary | ACGGAGAGC*tctaga*GACCGGCTG |
| | |

| Module 3 (pKOS024-2) | |
|---|---|
| 5' AT boundary | GACGGGCGC*ggatcc*GTCTTCCTG |
| AT/KR boundary | CGCTACTGG*ctgcag*CCCGCCGCA |
| 3' KR boundary | ACCGGCGAG*tctaga*CAACGGCTC |
| | |

| Module 4 (pKOS024-3) | |
|---|---|
| 5' AT boundary | GCGCCGCGC*ggatcc*GTCCTGGTC |
| AT(DH/ER/KR) boundary | CGCTTCTGG*ctgcag*CCGCACCGG |
| 3' DH/ER/KR boundary | GGGCCGAAC*tctaga*GACCGGCTC |
| | |

| Module 5 (pKOS006-182) | |
|---|---|
| 5' AT boundary | ACTCGCCGC*ggatcc*GCGATGGTG |
| AT/KR boundary | CGGTACTGG*ctgcag*ATCCCCACC |
| 3' KR boundary | GAGGAGGGC*tctaga*CTCGCCCAG |
| | |

| Module 6 (pKOS015-52) | |
|---|---|
| 5' AT boundary | TCCGCCGGC*ggatcc*GTTTTCGTC |
| AT/KR boundary | CGGTACTGG*ctgcag*CCGGAGGTG |
| 3' KR boundary | GTGGGGGCC*tctaga*GCGGTGCAG |

### Example 2

### Preparation of Cassettes from the Rapamycin PKS

A cosmid library of genomic DNA from *Streptomyces hygroscopicus* ATCC 29253 was used to prepare DNA cassettes prepared from the rapamycin PKS gene cluster to be used as replacements into the enzymatic activity regions of the erythromycin gene cluster. Cassettes were prepared by PCR amplification from appropriate cosmids or subclones using the primer pairs listed in Table 1, and were designed to introduce suitable restriction sites at the ends of the cassettes. The rapAT2 cassette is flanked by *Bgl*II and *Pst*I sites, and the rapAT14 cassette is flanked by *Bam*HI and *Pst*I sites. The reductive cycle cassettes are flanked by *Pst*I and *Xba*I sites. Large DH/ER/KR cassettes were amplified in two pieces, then joined at an engineered *Xho*I site in order to minimize errors introduced during PCR amplification of long DNA sequences. The rapKR4 cassette was made by cloning a 1.3kb *Nhe*I/*Xba*I fragment from the rapDH/KR4 cassette above into the *Xba*I site in pUC19. There is a *Pst*I site that is in-frame and upstream of *Xba*I in pUC19 that generates the following junction at the 5'-end of the cassette:
5'-*ctgcag*GTCGACTCTAGCCTGGT... (SEQ ID NO:23)

| **Table 1** | | |
|---|---|---|
| **Primer pairs used for PCT amplification of rapamycin PKS cassettes. All primers are listed from 5' to 3'. Engineered restriction sites are lower case.** | | |
| **Module** | **Primer** | **Sequence (SEQ ID NOS:24-35)** |
| rapAT2 | forward: | TTT*agatct*GTGTTCGTCTTCCCGGGT |
| | Reverse: | TT*ctgcag*CCAGTACCGCTGGTGCTGGAAGGCGTA |
| rapAT14 | Forward: | TTT*ggatcc*GCCTTCCTGTTCGACGGGCAAGGC |
| | Reverse: | TTT*ctgcag*CCAGTAGGACTGGTGCTGGAACGG |
| rapKR2 | Forward: | TTT*ctgcag*GAGGGCACGGACCGGGCGACTGCGGGT |
| | Reverse: | TTT*tctaga*ACCGGCGGCAGCGGCCCGCCGAGCAAT |
| rapDH/KR4 | Forward: | TT*ctgcag*AGCGTGGACCGGGCGGCT |
| | Reverse: | TTT*tctaga*GTCACCGGTAGAGGCGGCCCT |
| rapDH/ER/KR1 (left half) | Forward: | TTT*ctgcag*GGCGTGGACCGGGCGGCTGCC |
| | Reverse: | TTT*ctcgag*CACCACGCCCGCAGCCTCACC |
| rapDH/ER/KR1 (right half) | Forward: | TTT*ctcgag*GTCGGTCCGGAGGTCCAGGAT |
| | Reverse: | TTT*tctaga*ATCACCGGTAGAAGCAGCCCG |

### Example 3

### Replacement of DEBS Modules By Rapamycin PKS Cassettes

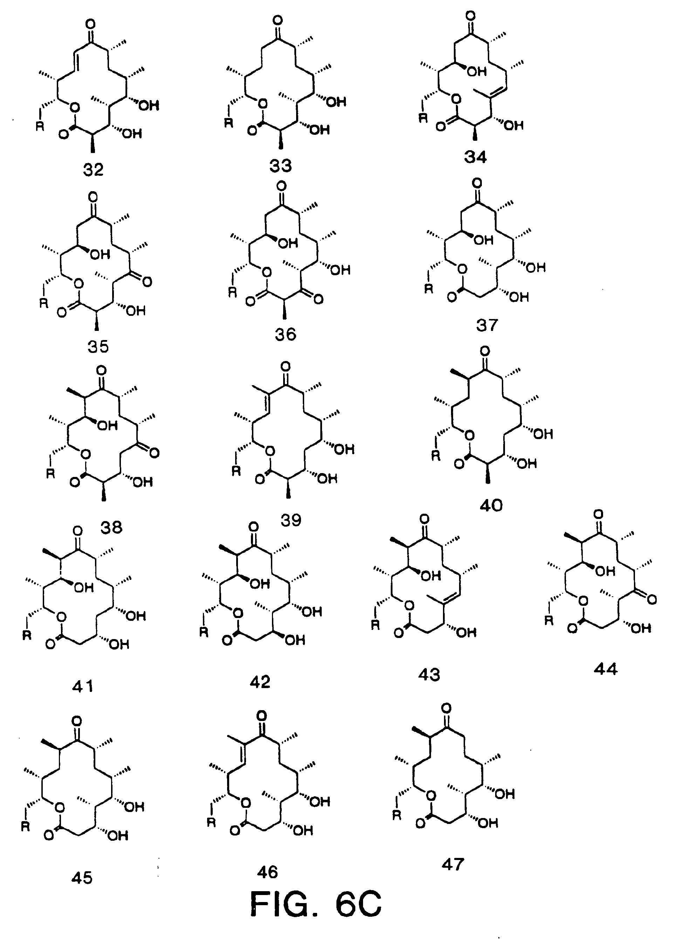

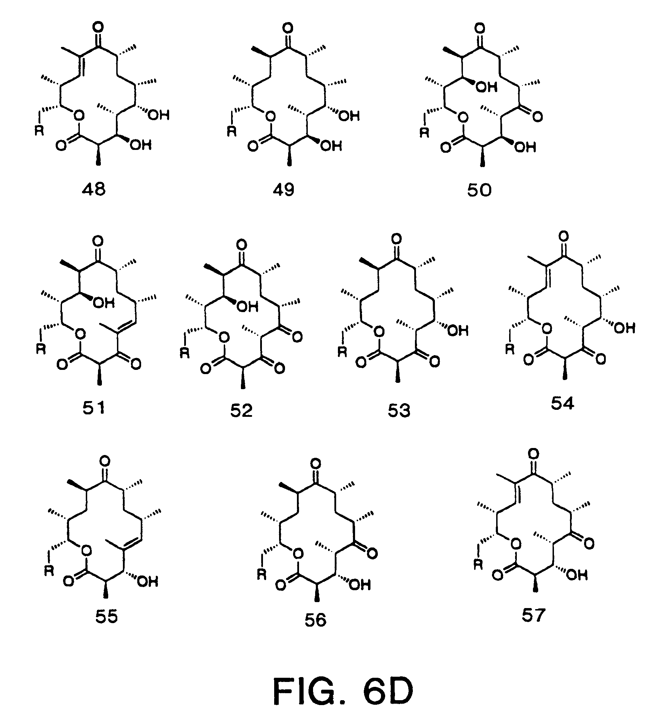

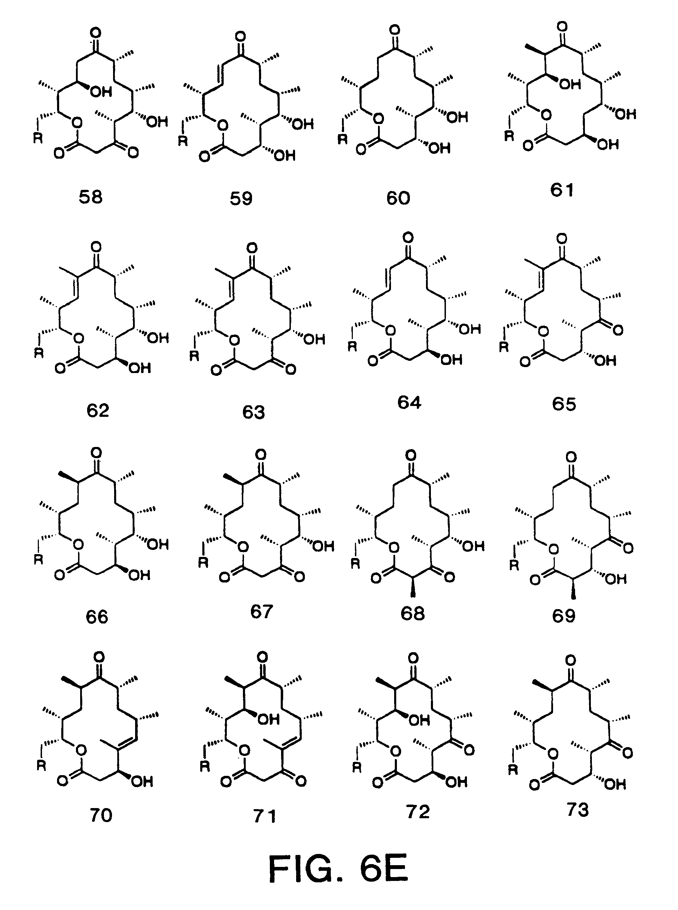

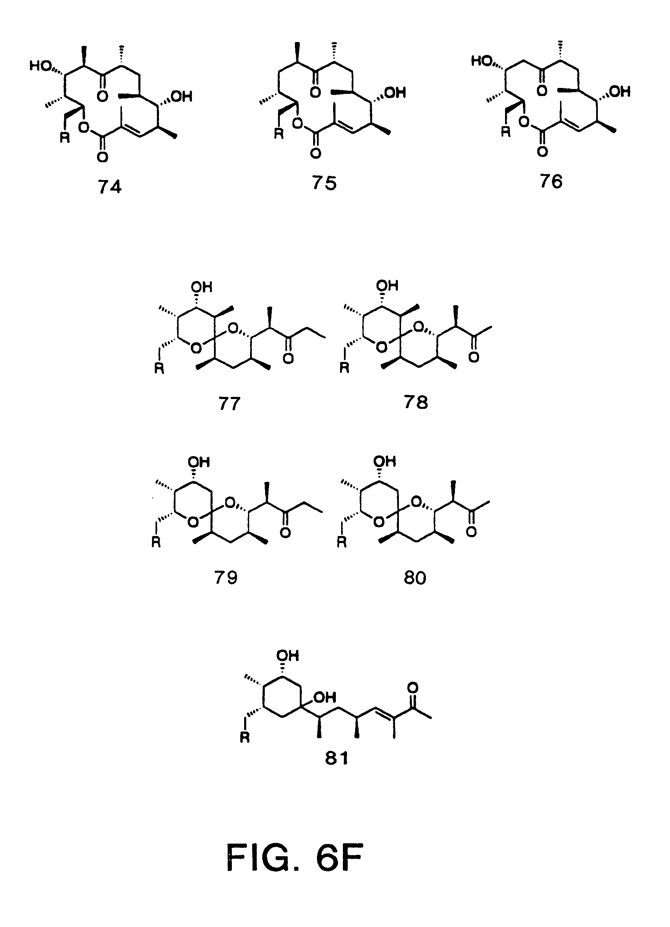

The following are typical procedures. The products are indicated by their numbers in Figure 6, where R may be methyl or hydrogen. In notations by these numbers in the tables and elsewhere, "a" represents R=methyl and "b" represents R=hydrogen.
a) Replacement of DEBS DH/ER/KR4. A portion of the erythromycin gene of module 4 (eryDH/ER/KR4) was replaced either with the corresponding rapamycin activities of the first rapamycin module (rapDH/ER/KR1) or of module 4 of rapamycin (rapDH/KR4). The replacement utilized the technique of Kao et al. Science (1994) 265:509-512. A donor plasmid was prepared by first amplifying 1 kbp regions flanking the DH/ER/KR4 of DEBS to contain a *Pst*I site at the 3' end of the left flank and an *Xba*I site at the 5' end of the right flank. The fragments were ligated into a temperature-sensitive donor plasmid, in a manner analogous to that set forth for KR6 in paragraph b) of this example, and the rapamycin cassettes prepared as described in Example 2 were inserted into the *Pst*I/*Xba*I sites. The recipient plasmid was pCK7 described in Preparation A. The *in vivo* recombination technique resulted in the expression plasmid pKOS011-19 (eryDH/ER/KR4 → rapDH/ER/KR1) and pKOS011-21 (eryDH/ER/KR4 → rapDH/KR4). The junctions at which the *Pst*I and *Xba*I sites were introduced into DEBS in both vectors are as follows:
**GAGCCCCAGCGGTACTGGCTGCAG rap cassette**
**TCTAGAGCGGTGCAGGCGGCCCCG (SEQ ID NOS:36-37)**

The resulting expression vectors were transformed into *S*. *coelicolor* CH999 and successful transformants grown as described above. The transformant containing the rapDH/ER/KR1 cassette produced the polyketide shown in Figure 6 as 23 where R is methyl or H; the transformant containing the plasmid with rapDH/KR4 cassette produced the polyketide shown in Figure 6 as 24 where R is methyl or H. As shown, these polyketides differ from 6-deoxyerythronolide B by virtue of a 6,7 alkene in the case of 24 where R is methyl and by the C6-methyl stereochemistry in the case of 23 where R is methyl.
b) Replacement of DEBS KR6. In a manner analogous to that set forth in paragraph a), plasmid pKOS011-25, wherein eryKR6 was replaced by rapDH/KR4. was prepared by substituting regions flanking the KR6 domain of DEBS in construction of the donor plasmid.
Approximately 1 kb regions flanking the eryKR6 domain were PCR amplified with the following primers (SEQ ID NOS:38-41) :

| | | |
|---|---|---|
| **left flank** | forward | 5'-TTTGGATCCGTTTTCGTCTTCCCAGGTCAG |
| | reverse | 5'-TTTCTGCAGCCAGTACCGCTGGGGCTCGAA |
| **right flank** | forward | 5'-TTTTCTAGAGCGGTGCAGGCGGCCCCGGCG |
| | reverse | 5'-AAAATGCATCTATGAATTCCCTCCGCCCA |

These fragments were then cloned into a pMAK705 derivative in which the multiple cloning site region was modified to accommodate the restriction sites of the fragments (i.e., *Bam*HI/*Pst*I for the left flank and *Xba*I*lNsi*I for the right flank). Cassettes were then inserted into the *Pst*I/*Xba*I sites of the above plasmid to generate donor plasmids for the *in vivo* recombination protocol. The resulting *Pst*I and *Xba*I junctions engineered into DEBS are as follows:
**GAACACCAGCGCTTCTGGCTGCAG rap cassette**
**TCTAGAGACCGGCTCGCCGGTCGG (SEQ ID NOS:42-43)**

Regions flanking the KR6 domain of DEBS were used to construct the donor plasmids.
Transformants of *S*. *coelicolor* CH999 resulted in the production of the polyketide shown in Figure 6 as 74 where R is methyl or H.
c) Replacement of DEBS KR2. The eryKR2 enzymatic activity was replaced in a series of vectors using *in vitro* insertion into the *Pst*I*lXba*I sites of pKAO263. pKAO263 is a derivative of pCK13 described in Kao, C.M. J Am Chem Soc (1996) 118:9184-9185. It was prepared by introducing the *Pst*I and *Xba*I restriction sites positioned identically to those in the analogous 2-module DEBS system described by Bedford, D. et al. Chem an Biol (1996) 3:827-831. Three expression plasmids were prepared: pKOS009-7 (eryKR2 → rapDH/KR4); pKAO392 (eryKR2 → rapKR2); and pKAO410 (eryKR2 → rapDH/ER/KR1). These plasmids, when transformed into *S*. *coelicolor* CH999, resulted in the production of polyketides with the structures 12, 3 and 10, where R is H or methyl and 10 where R is methyl in Figure 6, respectively. An additional vector, pKAO400 (eryKR2 → rapKR4) produced the same results as pKAO392.
d) Replacement of DEBS AT2. The DEBS AT activity from module 2 was excised after inserting restriction sites *Bam*HI and *PstI* flanking the AT module 2 domain into pCK12 (Kao et al. J Am Chem Soc (1995) 112:9105-9106). After digestion with *Bam*HI/*Pst*I, the *Bgl*II/*Pst*I fragment containing rapAT2 was inserted. The resulting engineered DEBS/rapAT2 junction is as follows (*Bam*HI/*Bgl*II ligation - GGATCT; *Pst*I *-* CTGCAG):
**AGTGCCTCCGACGGTGGATCT rapAT2 CTGCAGCCGGACCGCACCACCCCT (SEQ ID NOS:44-45)**

*S. coelicolor* CH999 transformed with the resulting plasmid, pKOS008-51, produced the polyketides 6 where R is methyl or H shown in Figure 6.

### Example 4

### Excision of DEBS Reductive Cycle Domains

The following is a typical procedure. The products are indicated by numbers, and the structures represented by the numbers are shown in Figure 6, where R can be either methyl (a) or hydrogen (b) as noted below.

A duplex oligonucleotide linker (ΔRdx) was designed to allow complete excision of reductive cycle domains. Two synthetic oligonucleotides (SEQ ID NOS:46-47): 
5'-GCCGGACCGCACCACCCCTCGTGACGGAGAACCGGAGACGGAGAGCT-3'
3'-ACGTCGGCCTGGCGTGGTGGGGAGCACTGCCTCTTGGCCTCTGCCTCTCGAGATC-5'
PstI XbaI
 were designed to generate *Pst*I*-* and *Xba*I-compatible ends upon hybridization. This duplex linker was ligated into the *Pst*I*-* and *Xba*I-sites of the recombination donor plasmid containing the appropriate left- and right-flanking regions of the reductive domain to be excised. The *in vivo* recombination technique of Example 3, paragraph a) was then used. The donor plasmid contained the duplex linker ΔRdx having a *Pst*I and *Xba*I compatible end ligated into the *Pst*I and *Xba*I sites of the plasmid modified to contain the left and right flanking regions of the reductive domain to be excised. The donor plasmids were recombined with recipient plasmid pCK7 to generate pKOS011-13 (eryKR6 → ΔRdx) and with recipient plasmid pCK13 to obtain pKOS005-4 (eryKR2 → ΔRdx). When transformed into *S*. *coelicolor* CH999, plasmid pKOS011-13 produced the polyketides 30, 31, 77 and 78 where R is methyl or H in each case; plasmid pKOS005-4 produced the polyketide 2 where R is methyl or H (Figure 6).

### Example 5

### Manipulation of Macrolide Ring Size by Directed Mutagenesis of DEBS

The following are typical procedures. The products are indicated by numbers, and the structures represented by the numbers are shown in Figure 6, where R can be either methyl (a) or hydrogen (b) as noted below.

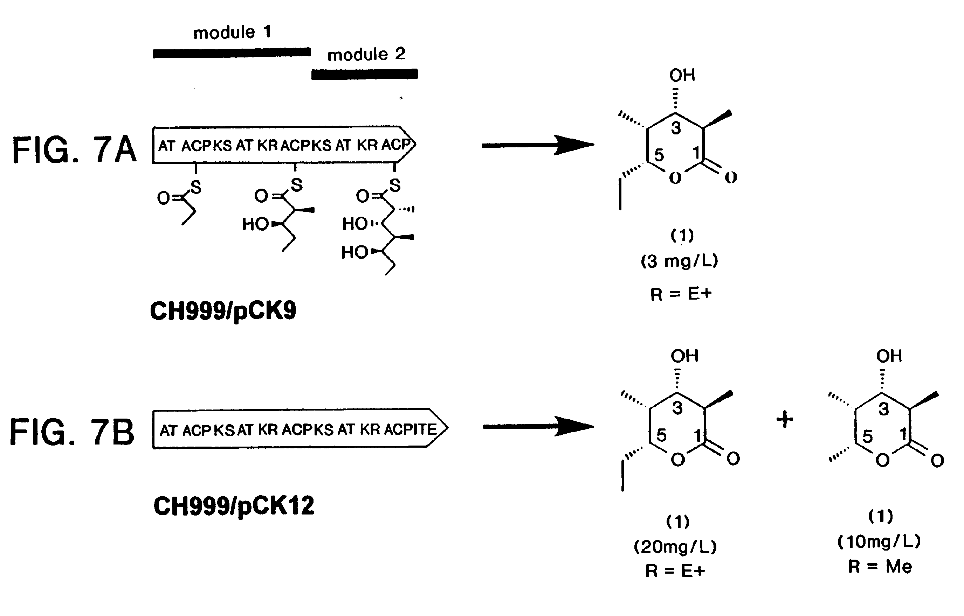

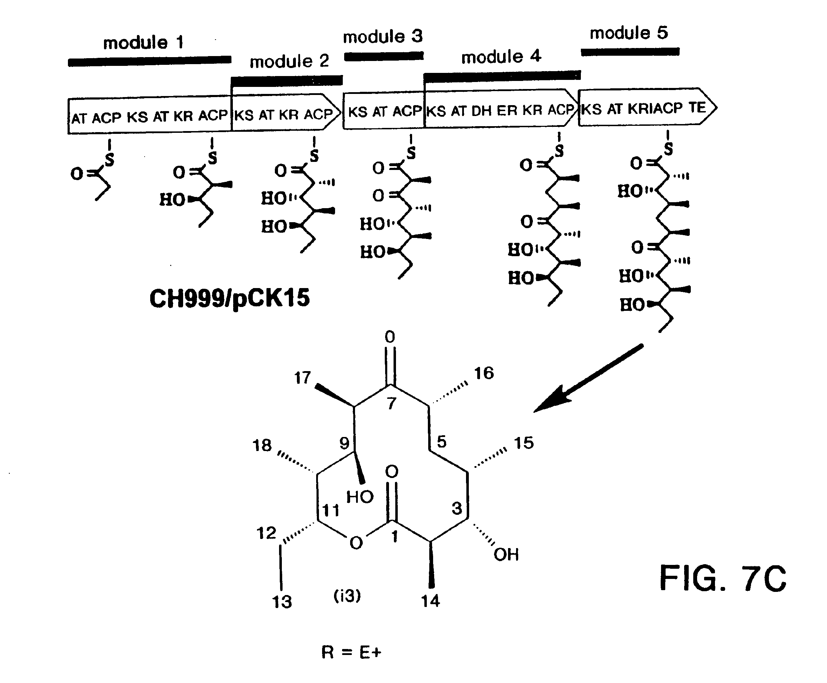

Using the expression system of Kao, C. M. et al. Science (1994) 265:509-512, the expression of DEBS1 alone (1 + 2), in the absence of DEBS2 and DEBS3 (in plasmid pCK9), resulted in the production of (2*R*,3*S*,4*S*,5*R*)-2,4-dimethyl-3,5-dihydroxy-*n*-heptanoic acid L-lactone ("the heptanoic acid L-lactone" (compound 1 in Figure 6, where R is methyl; also see Figure 7)) (1-3 mg/L), the expected triketide product of the first two modules (Kao, C. M. et al. J Am Chem Soc (1994) 116:11612-11613). Thus, a thioesterase is not essential for release of a triketide from the enzyme complex.

Two additional deletion mutant PKS were constructed. The first contained DEBS1 fused to the TE, and the second PKS included the first five DEBS modules fused to the TE (Figures 7B and 7C). Plasmids pCK52 and pCK15 respectively contained the genes encoding the bimodular ("1+2+TE") and pentamodular (" 1 +2+3+4+5+TE") PKSs.

The 1+2+TE PKS in pCK12 contained a fusion of the carboxy-terminal end of the acyl carrier protein of module 2 (ACP-2) to the carboxy-terminal end of the acyl carrier protein of module 6 (ACP-6). Thus ACP-2 is essentially intact and is followed by the amino acid sequence naturally found between ACP-6 and the TE. Plasmid pCK12 contained *ery*A DNA originating from pS1 (Tuan, J. S. et al. Gene (1990) 90:21). pCK12 is identical to pCK7 (Kao et al. Science (1994), *supra*) except for a deletion between the carboxy-terminal ends of ACP-2 and ACP-6. The fusion occurs between residues L3455 of DEBS1 and Q2891 of DEBS3. An *Spe*I site is present between these two residues so that the DNA sequence at the fusion is CTCACTAGTCAG (SEQ ID NO:48).

The 1+2+3+4+5+TE PKS in pCK15 contained a fusion 76 amino acids downstream of the β-ketoreductase of module 5 (KR-5) and five amino acids upstream of ACP-6. Thus, the fusion occurs towards the carboxy-terminal end of the non-conserved region between KR-5 and ACP-5, and the recombinant module 5 was essentially a hybrid between the wild type modules 5 and 6. Plasmid pCK15 contained *ery*A DNA originating from pS1 (Tuan et al. Gene (1990), *supra*). pCK15 is a derivative of pCK7 (Kao et al. Science (1994), *supra*) and was constructed using the *in vivo* recombination strategy described earlier (Kao et al. Science (1994), *supra*). pCK15 is identical to pCK7 with the exception of a deletion between KR-5 and ACP-6, which occurs between residues G1372 and A2802 of DEBS3, and the insertion of a blunted *Sal*I fragment containing a kanamycin resistance gene (Oka A. et al. J Mol Biol (1981) 147:217) into the blunted *Hind*III site of pCK7. An arginine residue is present between G1372 and A2802 so that the DNA sequence at the fusion is GGCCGCGCC.

Plasmids pCK12 and pCK15 were introduced into *S*. *coelicolor* CH999 and polyketide products were purified from the transformed strains according to methods previously described (Kao et al. Science (1994), *supra*)*.* The products obtained from various transformants: CH999/pCK12 and CH999/pCK15 as well as CH999/pCK9 described above, are shown in Figure 7.

CH999/pCK12 produced the heptanoic acid L-lactone (compound 1 in Figure 6, where R is methyl; also see Figure 7) (20 mg/L) as determined by ¹H and ¹³C NMR spectroscopy. This triketide product is identical to that produced by CH999/pCK9, which expresses the unmodified DEBS1 protein alone described above. However, CH999/pCK12 produced this compound in significantly greater quantities than did CH999/pCK9 (>10 mg/L vs. ~1 mg/L), indicating the ability of the TE to catalyze thiolysis of a triketide chain attached to the ACP domain of module 2. CH999/pCK12 also produced significant quantities of compound (R is H), a novel analog of compound (R is methyl), (10 mg/L), that resulted from the incorporation of an acetate start unit instead of propionate. This is reminiscent of the ability of CH999/pCK7, which expresses the intact PKS, to produce 8,8a-deoxyoleandolide (see Figure 1A) in addition to 6dEB described above.

Since compound 1 (R is H) was not detected in CH999/pCK9, its facile isolation from CH999/pCK12 provides additional evidence for the increased turnover rate of DEBS1 due to the presence of the TE. In other words, the TE can effectively recognize an intermediate bound to a "foreign" module that is four acyl units shorter than its natural substrate, 6dEB. However, since the triketide products can probably cyclize spontaneously into compound 1 where R is methyl or H under typical fermentation conditions (pH 7), it is not possible to discriminate between a biosynthetic model involving enzyme-catalyzed lactonization and one involving enzyme-catalyzed hydrolysis followed by spontaneous lactonization. Thus, the ability of the 1+2+TE PKS to recognize the C-5 hydroxyl of a triketide as an incoming nucleophile is unclear.

CH999/pCK15, produced abundant quantities of (8R,9S)-8,9-dihydro-8-methyl-9-hydroxy-10-deoxymethonolide (compound 13 in Figure 6) (10 mg/L), demonstrating that the pentamodular PKS is active. Compound 13 was characterized using ¹H and ¹³C NMR spectroscopy of natural abundance and ¹³C-enriched material, homonuclear correlation spectroscopy (COSY), heteronuclear correlation spectroscopy (HETCOR), mass spectrometry, and molecular modeling. Compound 13 is an analog of 10-deoxymethonolide (compound 14, Lambalot, R.H. et al. J Antibiotics (1992) 45:1981-1982), the aglycone of the macrolide antibiotic methymycin. The production of 13 by a pentamodular enzyme demonstrates that active site domains in modules 5 and 6 in DEBS can be joined without loss of activity. Thus, it appears that individual modules as well as active sites are independent entities which do not depend on association with neighboring modules to be functional. The 12-membered lactone ring, formed by esterification of the terminal carboxyl with the C-11 hydroxyl of the hexaketide product, indicated the ability of the 1+2+3+4+5+TE PKS, and possibly the TE itself, to catalyze lactonization of a polyketide chain one acyl unit shorter than the natural product of DEBS, 6dEB. Indeed, the formation of compound 13 may mimic the biosynthesis of the closely related 12-membered hexaketide macrolide, methymycin, which frequently occurs with the homologous 14-membered heptaketide macrolides, picromycin and/or narbomycin (Cane, D. E. et al. J Am Chem Soc (1993) 115:522-566). The erythromycin PKS scaffold can.thus be used to generate a wide range of macrolactones with shorter as well as longer chain lengths.

The construction of the 1+2+3+4+5+TE PKS resulted in the biosynthesis of a previously uncharacterized 12-membered macrolactone that closely resembles, but is distinct from, the aglycone of a biologically active macrolide. The apparent structural and functional independence of active site domains and modules as well as relaxed lactonization specificity suggest the existence of many degrees of freedom for manipulating these enzymes to produce new modular PKSs.

### Example 6

### Production and Analysis of Polyketide Products

The expression vectors created by domain substitution in DEBS, as described in Examples 1-5, were transformed into either *Streptomyces coelicolor* CH999 or *S. lividans* K4-114 using standard techniques (D.A. Hopwood et al. (1985) "Genetic Manipulation of Streptomyces: A Laboratory Manual," (The John Innes Foundation, Norwich)). Both host strains have complete deletions of the native actinorhodin polyketide synthase gene cluster and so produce no native polyketide products. Transformants were grown on 150 mm R2YE agar plates for 2 days at 30°C, at which time the agar slab was lifted from the dish and placed in a new dish which contained a layer of 4 mm glass beads, 50 mL of liquid R2YE medium supplemented with 5 mM sodium propionate, and ca. 1 g of XAD-16 resin beads. This was kept at 30°C for an additional 7 days.

The XAD-16 resin was collected by vacuum filtration, washed with water, then extracted twice with 10 mL portions of ethanol. The extracts were combined and evaporated to a slurry, which was extracted with ethyl acetate. The ethyl acetate was washed once with sat. NaHCO₃ and evaporated to yield the crude product. Samples were dissolved in ethanol and analyzed by LC/MS. The HPLC used a 4.6 x 150 mm C 18 reversed-phase column with a gradient from 80:19:1 H₂O/CH₃CN/CH₃CO₂H to 99:1 CH₃CN/CH₃CO₂H. Mass spectra were recorded using a Perkin-Elmer/Sciex API100LC spectrometer fitted with an APCI ion source. Each genetic construct typically resulted in formation of products in pairs, indicated in Table 2 by the letters "a" (R = methyl) and "b" (R = H), arising from priming of the PKS by propionyl-CoA and acetyl-CoA, respectively.

### Additional Examples

Using the foregoing techniques, the DEBS constructs shown in Table 2 were prepared. The products obtained when the constructs were transformed into *S. coelicolor* CH999 are indicated by their numbers in Figure 6, where R in embodiments designated by "a" in Table 2 represents methyl and in embodiments designated by "b" in Table 2 represents hydrogen. Some of the expression vectors were prepared by *in vitro* ligation; multiple domain substitutions were created by subsequent *in vitro* ligations into the singly-substituted expression plasmids. Others were obtained by *in vivo* recombination.

| **Table 2** | | | |
|---|---|---|---|
| **Plasmid** | **Modules** | **Domain Substitution** | **Products (see** **Figure 6****)** |
| ***In Vitro* Ligation** | | | |
| KOS011-28 | 2 | eryAT1 → rapAT2 | 4-nor-TKL (5a,b) |
| KOS008-51 | 2 | eryAT2 → rapAT2 | 2-nor-TKL (6a,b) |
| KOS014-62 | 2 | eryKR2 → rapDH/ER/KR1 | 3-deoxy-TKL (4a,b) |
| KAO410 | 3 | eryKR2 → rapDH/ERlKR1 | KAO410 (10a,b) 3-deoxy-hemiketal (11a,b) |
| KA0392 | 3 | eryKR2 → rapKR2 | 3-epi-TKL (3a,b) |
| KOS009-7 | 3 | eryKR2 → rapDH/KR4 | KOS009-7 (12a,b) |
| KOS015-30 | 6 | eryAT3 → rapAT2 | 8-nor-6dEB (18a,b) |
| KOS016-47 | 6 | eryAT5 → rapAT2 | 4-nor-6dEB (19a,b) |
| KOS026-18b | 6 | eryKR5 → rapDH/ER/KR1 | 5-deoxy-6dEB (26a,b) |
| KOS016-32 | 6 | eryKR5 → rapDH/KR4 | 4,5-dehydro-6dEB (27a,b) |
| KOS016-28 | 6 | eryKR5 → ΔRdx | 5-oxo-6dEB (28a,b) |
| KOS015-63 | 6 | eryAT6 → rapAT2 | 2-nor-6dEB (20a,b) |
| KOS015-83 | 6 | eryAT2 → rapAT2 + eryKR2 → rapDH/KR4 | 10-nor-10,11-dehydro-6dEB (32a,b) |
| KOS015-84 | 6 | eryAT2 → rapAT2 + eryKR2 → rapDH/ER/KR1 | 10-nor-11-deoxy-6dEB (33a, b) |
| KOS016-100 | 6 | eryAT5 → rapAT2 + eryKR5 → Δrdx | 4-nor-5-oxo-6dEB (38a,b) |
| KOS015-106 | 6 | eryAT6 → rapAT2 + eryKR6 → rapKR2 | 2-nor-3-epi-6dEB (42a,b) |
| KOS015-109 | 6 | eryAT6 → rapAT2 + eryKR6 → Δrdx | 2-nor-3-oxo-6dEB (31a,b) |
| KOS011-90 | 6 | eryAT2 → rapAT2 + eryKR5 → rapDH/KR4 | 4,5-dehydro-10-nor-6dEB (34a,b) |
| KOS011-84 | 6 | eryAT2 → rapAT2 + eryKR5 → Δrdx | 5-oxo-10-nor-6dEB (35a,b) |
| KOS011-82 | 6 | eryKR2 → rapDH/KR4 + eryAT5 → rapAT2 | 4-nor-10,11-dehydro-6dEB (39a,b) |
| KOS011-85 | 6 | eryKR2 → rapDH/KR4 + eryKR5 → Δrdx | 5-oxo-10,11-dehydro-6dEB (57a,b) |
| KOS011-87 | 6 | eryKR2 → rapDH/KR4 + eryAT5 → rapAT2 + eryKR5 → Δrdx | 4-nor-5-oxo-10,11-dehydro-6dEB (65a,b) |
| KOS011-83 | 6 | eryKR2 → rapDH/ER/KR1 + eryAT5 → rapAT2 | 4-nor-11-deoxy-6dEB (40a,b) |
| KOS011-91 | 6 | eryKR2 → rapDH/ER/KR1 + eryKR5 → rapDH/KR4 | 4,5-dehydro-11-deoxy-6dEB (55a,b) |
| KOS011-86 | 6 | eryKR2 → rapDH/ER/KR1 + eryKR5 → Δrdx | 5-oxo-11-deoxy-6dEB (56a,b) |
| KOS011-88 | 6 | eryKR2 → rapDH/ER/KR1 + eryAT5 → rapAT2 eryKR5 → Δrdx | 4-nor-5-oxo-11-deoxy-6dEB (69a,b) |
| KOS015-₋40 | 6 | eryAT2 → rapAT2 + eryKR6 → rapDH/KR4 | 2,3-dehydro-10-nor-6dEB (76a,b) |
| KOS015-41 | 6 | eryAT2 → rapAT2 + eryKR6 → Δrdx | 3-oxo-10-nor-6dEB (36a,b) 10-nor-spiroketal (79a,b) |
| KOS015-44 | 6 | eryKR2 → rapDH/ER/KR1 + eryAT6 → rapAT2 | 2-nor-11-deoxy-6dEB (45a,b) |
| KOS015-45 | 6 | eryKR2 → rapDH/ER/KR1 + eryKR6 → RapDH/KR4 | 2,3-dehydro-11-deoxy-6dEB (75a,b) |
| KOS015-46 | 6 | eryKR2 → rapDH/ER/KR1 + eryKR6 → Δrdx | 3-oxo-11-deoxy-6dEB (53a,b) |
| KOS015-42 | 6 | eryKR2 → rapDH/KR4 + eryAT6 → rapAT2 | 2-nor-10,11-dehydro-6dEB (46a,b) |
| KOS015-43 | 6 | eryKR2 → rapDH/KR4 + eryKR6 → Δrdx | 3-oxo-10,11-dehydro-6dEB (54a,b) |
| KOS015-88 | 6 | eryKR2 → rapDH/KR4 + eryKR6 → rapKR2 | 3-epi-10,11-dehydro-6dEB (48a,b) |
| KOS015-89 | 6 | eryKR2 → rapDH/ER/KR1 + eryKR6 → rapKR2 | 3-epi-11-deoxy-6dEB (49a,b) |
| KOS015-87 | 6 | eryAT2 → rapAT2 + eryKR6 → rapKR2 | 3-oxo-10-nor-6dEB (36a,b) |
| KOS015-117 | 6 | eryAT2 → rapAT14 + eryAT6 → rapAT2 | 2,10-bisnor-6-dEB (37a,b) |
| KOS015-120 | 6 | eryAT2 → rapAT14 + eryAT6 → rapAT2 + eryKR6 → Δrdx | 2,10-bisnor-3-oxo-6dEB (58a,b) |
| | | | 2,10-bisnor-spiroketal (80a,b) |
| KOS015-121 | 6 | eryKR2 → rapDH/KR4 + eryAT6 → rapAT2 + eryKR6 → rapKR2 | 2-nor-3-epi-10,11-dehydro-6dEB (62a,b) |
| KOS015-122 | 6 | eryKR2 → rapDH/KR4 + eryAT6 → rapAT2 + eryKR6 → Δrdx | 2-nor-3-oxo-10,11-dehydro-6dEB (63a,b) |
| KOS015-123 | 6 | eryKR2 → rapDH/ER/KR1 + eryAT6 → rapAT2 + eryKR6 → rapKR2 | 2-nor-3-epi-11-deoxy-6dEB (66a,b) |
| KOS015-125 | 6 | eryKR2 → rapDH/ER/KR1 + eryAT6 → rapAT2 + eryKR6 → Δrdx | 2-nor-3-oxo-11-deoxy-6dEB (67a,b) |
| KOS015-127 | 6 | eryAT2 → rapAT2 + eryKR2 → rapDH/KR4 + eryKR6 → rapKR2 | 3-epi-10-nor-10,11-dehydro-6dEB (64a,b) |
| KOS015-150 | 6 | eryAT2 → rapAT2 + eryKR2 → rapDH/KR4 + eryAT6 → rapAT2 | 2,10-bisnor-10,11-dehydro-6dEB (59a,b) |
| KOS015-158 | 6 | eryAT2 → rapAT2 + eryKR2 → rapDH/ER/KR1 + eryKR6 → Δrdx | 3-oxo-10-nor-11-deoxy-6dEB (68a,b) |
| KOS015-159 | 6 | eryAT2 → rapAT2 + eryKR2 → rapDH/ER/KR1 + eryAT6 → rapAT2 | 2,10-bisnor-11-deoxy-6dEB (60a,b) |
| KOS016-133K | 6 | eryKRS → rapDH/KR4 + eryKR6 → Δrdx | 3-oxo-4,5-dehydro-6dEB (51a,b) |
| | | | 3,5-dioxo-6dEB (52a,b) |
| KOS016-150B | 6 | eryKR5 → Δrdx + eryKR6 → rapKR4 | 3-epi-5-oxo-6dEB (50a,b) |
| KOS016-183F | 6 | eryAT5 → rapAT2 + eryAT6 → rapAT2 | 2,4-bisnor-6dEB (41a,b) |
| KOS016-183G | 6 | eryAT5 → rapAT2 + eryAT6 → rapAT2 + eryKR6 → rapKR2 | 2,4-bisnor-3-epi-6dEB (61a,b) |
| KOS016-152E | 6 | eryKR5 → rapDH/KR4 + eryAT6 → rapAT2 | 2-nor-4,5-dehydro-6dEB (43a,b) |
| KOS016-152F | 6 | eryKR5 → rapDH/KR4 + eryAT6 → rapAT2 + eryKR6 → rapKR2 | 2-nor-3-epi-4,5-dehydro-6dEB (70a,b) |
| KOS016-152G | 6 | eryKR5 → rapDH/KR4 + eryAT6 → rapAT2 + eryKR6 → Δrdx | 2-nor-3-oxo-4,5-dehydro-6dEB (71a,b) |
| | | | hemiketal (81a,b) |
| KOS016-152K | 6 | eryKR5 → Δrdx + eryAT6 → rapAT2 | 2-nor-5-oxo-6dEB (44a,b) |
| KOS016-152I | 6 | eryKR5 → Δrdx + eryAT6 → rapAT2 + eryKR6 → rapKR2 | 2-nor-3-epi-5-oxo-6dEB (72a,b) |
| KOS015-34 | 6 | eryAT3 → rapAT2 + eryAT6 → rapAT2 | 2,8-bisnor-6dEB (47a,b) |
| KOS015-162 | 6 | EryKR2 → rapDH/ER/KR1 + eryKR5 → Δrdx + eryAT6 → rapAT2 | 2-nor-5-oxo-11-deoxy-6dEB (73a,b) |

| *In Vivo* Ligation | | | |
|---|---|---|---|
| KOS005-4 | 3 | KR2 → ΔRdx | 3-keto-TKL (2a,b) |
| KOS011-62 | 6 | AT2 → rapAT2 | 10-nor-6dEB (17a,b) |
| KOS011-66 | 6 | KR2 → rapDH/ER/KR1 | 11-deoxy-6dEB (21a,b) |
| KOS011-64 | 6 | KR2 → rapDH/KR4 | 10,11-dehydro-6dEB (22a,b) |
| KOS011-19 | 6 | DH/ER/KR4 → rapDH/ER/KR1 | 6-epi-6dEB (23a,b) |
| KOS011-21 | 6 | DH/ER/KR4 → rapDH/KR4 | 6,7-dehydro-6dEB (24a,b) |
| KOS011-22 | 6 | DH/ER/KR4 → ΔRdx | 7-oxo-6dEB (25a,b) |
| KOS011-74 | 6 | KR6 → rapKR2 | 3-epi-6dEB (29a,b) |
| KOS011-25 | 6 | KR6 → rapDH/KR4 | 2,3-dehydro-6dEB (74a,b) |
| KOS011-13 | 6 | KR6 → ΔRdx | 3-oxo-6dEB (30a,b) |
| | | | 2-nor-3-oxo-6dEB(31a,b) |
| | | | spiroketal (77a,b) |
| | | | 2-nor-spiroketal (78a,b) |

### Example 7

### Preparation of 14,15-dehydro-6-deoxyerythronolide B (Compound 94 of Figure 9)

A 3 day culture of S. coelicolor CH999/pJRJ2 grown on 3 100-mm R2YE agar plates was overlayed with a solution of 10 mg of (2S,3R)-3-hydroxy-2-methyl-4-pentenoic acid N-acetylcysteamine thioester dissolved in 2 mL of 9:1 water/DMSO and allowed to dry. The culture was incubated at 30°C for an additional 4 days. The agar was chopped and extracted twice with an equal volume of ethyl acetate. The extracts were combined and evaporated. Purification by silica gel chromatography (1:1 ethyl acetate/hexanes) yielded 0.75 mg of 14,15-dehydro-6-deoxyerythronolide B, compound 94 in Figure 9, APCI-MS gives [M+H]+ = 385.

Analogous compounds with variations in R* and/or R¹ as represented by compounds 96-107 and compound 113 of figures 6G and 6H are prepared in a similar manner as described in the previous paragraph but substituting the appropriate diketide as the N-acetylcysteamine thioester. These compounds are prepared in this manner and their structures verified.

The preparation of the appropriate derivatized diketides is described in Example 17.

### Example 8

### Synthesis of 1-(2-mercaptopyrimidinyl)-2-O-methoxycarbonyl-(D)-desosamine

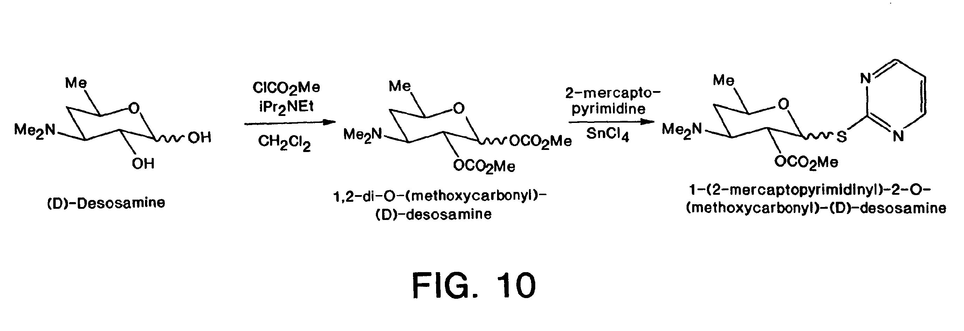

For the glycosylation reactions in the following examples, the title compound was used as a reagent. The conversions of paragraph (A) and (B) of this Example are shown in Figure 10.
(A) Preparation of 1,2-di-O-methoxycarbonyl-(D)-desosamine: To 1.00 g of (D)-desosamine (4.74 mmol) in 50 mL CH₂Cl₂ was added 3.06 g of diisopropylethylamine. The mixture was stirred at ambient temperature for 10 min, then cooled to 4°C. Methyl chloroformate (1.34 g) was added dropwise at 4°C. The reaction mixture was allowed to warm to ambient temperature and stirred overnight. The solvent was evaporated to dryness, ethyl acetate (150 mL) was added to extract the product, and the remaining solid was filtered. The ethyl acetate was removed under vacuum and the crude product was purified on a silica gel column (ethyl acetate:methanol:triethylamine 84:5:1 v/v/v) to give 1.29 g of product (88% yield).
(B) Preparation of 1-(2-mercaptopyrimidinyl)-2-O-methoxycarbonyl-(D)-desosamine: A mixture of 1,2-di-O-methoxycarbonyl-(D)-desosamine (1.00 g, 3.436 mmol) and 0.7697 g of 2-mercaptopyrimdine (6.872 mmol) in a 25 mL 2-neck flash is dried under vacuum for 45 minutes. Dichloroethane (10 mL), toluene (5 mL), and DMF (5 mL) were added and stirred at ambient temperature followed by addition of 7 mL of SnCl₄ (1M in CH₂Cl₂). The reaction mixture was kept at 80°C overnight. The reaction was terminated by addition of 1N NaOH until the mixture turned basic. The solution was extracted with 300 mL of ethyl acetate and the organic layer was washed with saturated aqueous NaHCO₃ (3 x 150 mL), dried over Na₂SO₄, filtered. and evaporated. The product was purified on a silica gel column (1:1 ethyl acetate:hexanes to ethyl acetate with 1% triethylamine) to obtain 0.25 g of 1-(2-mercaptopyrimidinyl)-2-O-methoxycarbonyl-(D)-desosamine and 0.5 g of recovered 1,2-di-O-methoxycarbonyl-(D)-desosamine.

### Example 9

### Preparation of 5-O-[1-β-(2-O-methoxycarbonyl-(D)-desosaminyl)]-6-deoxyerythronolide B and 5-O-(1-β-(D)-desosaminyl)-deoxyerythronolide B (Compounds 86 and 87 in Figure 8)

(A) A mixture of 6-deoxyerythronolide B (6-DEB) (15 mg, 39 umol) and 1-(2-mercaptopyrimidinyl)-2-O-methoxycarbonyl-(D)-desosamine (65 mg, 200 umol) was dried under vacuum, then placed under a nitrogen atmosphere. To this was added CH₂Cl₂ (1 mL), toluene (0.5 mL), and powdered 4A molecular sieves (50 mg), and the mixture was stirred for 10 minutes at ambient temperature. Silver trifluoromethanesulfonate (64 mg, 250 umol) was added and the reaction was stirred until LC/MS analysis indicated completion (18-20 hours). The mixture was filtered through anhydrous Na₂SO₄ and evaporated to yield crude product. The residue was dissolved in several drops of acetonitrile and loaded on a C-18 solid phase extraction cartridge (Whatman). Unreacted desosamine was removed by washing with 20% CH₃CN/H₂O and glycosylation products and the remaining macrolide aglycone were recovered by eluting with 100% CH₃CN. Final separation was carried out by HPLC using a semiprep C-18 column (10 mm X 150 mm) (CH₃CN/H₂O 20% isocratic over 5 min, then 20% to 80% over 30 min). HPLC fractions were checked by LC/MS and fractions containing the same product were combined. The solvent was removed under vacuum, yielding 8.4 mg of 5-O-[1-β-(2-O-methoxycarbonyl-(D)-desosaminyl)]-6-deoxyerythronolide B (compound 86 in Figure 8) (36% yield). APCI-MS gives [M+H]+ = 602.
(B) 5-O-[1-(2-O-methoxycarbonyl-(D)-desosaminyl)]-6-deoxyerythronolide B (1-6 mg) from paragraph (A) was dissolved in 1 mL methanol, 0.2 mL H₂O, and 0.2 mL triethylamine and kept at 70°C for 3 hours. Removal of the solvent under vacuum gave crude product. This was dissolved in a few drops of CH₃CN and applied to a Whatman C18 solid phase extraction cartridge. The column was washed with 25 mL of 20% CH₃CN in water, then the product was eluted with 100% CH₃CN. Evaporation of the solvent gave 5-O-(1-β-(D)-desosaminyl)-6-deoxyerythronolide B (compound 87 in Figure 8) in quantitative yield. APCI-MS gives [M+H]+ = 544.

### Example 10

### Preparation of 5-O-[1-β-(2-O-methoxycarbonyl-(D)-desosaminyl)]-8,8a-deoxyoleandolide and 5-O-(1-β-(D)-desosaminyl)]-8,8a-deoxyoleandolide (Compounds 88 and 89 in Figure 8)

(A) Treatment of 8,8a-deoxyoleandolide (12 mg) as described in Example 9(A) yielded 5-O-[1-β-(2-O-methoxycarbonyl-(D)-desosaminyl)]-8,8a-deoxyoleandolide (60% yield) (compound 88 in Figure 8). APCI-MS gives [M+H]+ = 508.
(B) Treatment of 5-O-[1-β-(2-methoxycarbonyl-(D)-desosaminyl)]-8,8a-deoxyoleandolide of paragraph (A) as described in Example 9(B) gave 5-O-(1-β-(D)-desosaminyl)-8,8a-deoxyoleandolide (compound 89 in Figure 8) in quantitative yield. APCI-MS gives [M+H]+ = 530.

### Example 11

### Preparation of 5-O-[1-β-(2-methoxycarbonyl-(D)-desosaminyl)]-3,6-dideoxy-3-oxoerythronolide B (Compound 90 in Figures 8) and 5,11-bis-(O-[1-β-(2-methoxycarbonyl-(D)-desosaminyl)])-3,6-dideoxy-3-oxoerythronolide B (Compound 92 in Figure 8)

Treatment of 3,6-dideoxy-3-oxoerythronolide B (6 mg) as described in Example 9(A) gave 5-O-[1-β-(2-O-methoxycarbonyl-(D)-desosaminyl)]-3,6-dideoxy-3-oxoerythronolide B (compound 90 in Figure 8) in 44% yield. APCI-MS gives [M+H]+ = 600. A second product, 5,11-bis-(O-[1-β-(2-O-methoxycarbonyl-(D)-desosaminyl)])-3,6-dideoxy-3-oxoerythronolide B (compound 92 in Figure 8), was also isolated from this mixture in 26% yield; APCI-MS gives [M+H]+ = 815.

### Example 12

### Preparation of 5-O-(1-β-(D)-desosaminyl)-3,6-dideoxy-3-oxoerythronolide B (Compound 91 in Figure 8) and of 5,11-bis-O-(1-β-(D)-desosaminyl)-3,6-dideoxy-3-oxoerythronolide B (Compound 93 in Figure 8)

Treatment of 5-O-[1-β-(2-methoxycarbonyl-(D)-desosaminyl)]-3,6-dideoxy-3-oxoerythronolide B of Example 11 as described in Example 9(B) gave 5-O-(1-β-(D)-desosaminyl)-3,6-dideoxy-3-oxoerythronolide B (compound 91 in Figure 8) in quantitative yield. APCI-MS gives [M+H]+ = 542.

Treatment of 5,11-bis-(O-[1-β-(2-methoxycarbonyl-(D)-desosaminyl)])-3,6-dideoxy-3-oxoerythronolide B of Example 11 as described in Example 9(B) gave 5,11-bis-O-(1-β-(D)-desosaminyl)-3,6-dideoxy-3-oxoerythronolide B (compound 93 in Figure 8) in quantitative yield. APCI-MS gives [M+H]+ = 699.

### Example 13

### Preparation of 2'-O-methoxycarbonyl-(8R,9S)-10-deoxy-8,9-dihydro-9-hydroxy-8-methylmethymycin (Compound 82 in Figure 8) and 3,9-bis-(O-[1-β-(2-methoxycarbonyl-(D)-desosaminyl)])-(8R,9S)-10-deoxy-8,9-dihydro-9-hydroxy-8-methylmethonolide (Compound 84 in Figure 8)

Treatment of (8R,9S)-10-deoxy-8,9-dihydro-9-hydroxy-8-methylmethymycin (12 mg) according to the procedure of Example 9(A) yielded 2'-O-methoxycarbonyl-(8R,9S)-10-deoxy-8,9-dihydro-9-hydroxy-8-methylmethymycin (compound 82 in Figure 8) (34%); APCI-MS gave [M+H]+= 544. A second product, 3,9-bis-(O-[1-β-(2-O-methoxycarbonyl-(D)-desosaminyl)])-(8R,9S)-10-deoxy-8,9-dihydro-9-hydroxy-8-methylmethonolide (compound 84 in Figure 8), was also isolated from this mixture (33%); APCI-MS gave [M+H]+ = 759.

### Example 14

### Preparation of (8R,9S)-10-deoxy-8,9-dihydro-9-hydroxy-8-methylmethymycin (Compound 83 in Figure 8) and of (8R,9S)-10-deoxy-8,9-dihydro-9-(1-β-(D)-desosaminyloxy)-8-methylmethymycin (Compound 85 in Figure 8)

Treatment of 2'-O-methoxycarhonyl-(8R,9S)-10-deoxy-8,9-dihydro-9-hydroxy-8-methylmethymycin of Example 13 as described in Example 9(B) gave (8R,9S)-10-deoxy-8,9-dihydro-9-hydroxy-8-methylmethymycin (compound 83 in Figure 8) in quantitative yield. APCI-MS gives [M+H]+ = 486.

Treatment of 3,9-bis-(O-[1-β-(2-methoxycarbonyl-(D)-desosaminyl)])-(8R,9S)-10-deoxy-8,9-dihydro-9-hydroxy-8-methylmethonolide of Example 13 as described in Example 9(B) gave (8R,9S)-10-deoxy-8,9-dihydro-9-hydroxy-8-methylmethymycin (compound 85 in Figure 8) in quantitative yield (elution from the C 18 solid-phase extraction cartridge was with 100% methanol). APCI-MS gives [M+H]+=643.

### Example 15

### Preparation of 14,15-dehydroerythromycin A (Compound 95 in Figure 9)

A sample of 14,15-dehydro-6-deoxyerythronolide B (0.75 mg) from Example 7 was dissolved in 0.6 mL of ethanol and diluted to 3 mL with sterile water. This solution was used to overlay a 3 day old culture of *Saccharopolyspora erythraea* WHM34 (eryA) grown on a 100 mm R2YE agar plate at 30°C. After drying, the plate was incubated at 30°C for 4 days. The agar was chopped and extracted 3 times with 100 mL portions of 1% triethylamine in ethyl acetate. The extracts were combined and evaporated. The crude product was purified by preparative HPLC (C 18 reversed phase, water-acetonitrile gradient containing 1% acetic acid). Fractions were analyzed by mass spectrometry, and those containing pure 14,15-dehydroerythromycin A (compound 95 in Figure 9) were pooled, neutralized with triethylamine, and evaporated to a syrup. This was dissolved in water and extracted 3 times with equal volumes of ethyl acetate. The organic extracts were combined, washed once with saturated aqueous NaHCO₃, dried over Na₂SO₄, filtered, and evaporated to yield 0.15 mg of product. APCI-MS gives [M+H]+ = 733.

### Example 16

### Preparation of 14-oxo-8,8a-deoxyoleandolide (Compound 108) and 8,8a-deoxyoleandolide-14-carboxylic acid (compound 109) and Derivatives Thereof

These compounds can be prepared through ozonolysis of 14,15-dehydro-6-deoxyerythonolide B (compound 94 of Figure 9).

A solution of compound 94 in methanol is cooled to -40°C, and ozone is bubbled into the solution until formation of I₂ is observed in a KI solution attached to the outlet of the reaction vessel. Excess ozone is purged from the solution by sparging with nitrogen gas, providing a solution of the ozonide of compound 94. Treatment of this solution with Me₂S will reduce the ozonide to the aldehyde, compound 108.

Alternatively, the ozonide can be oxidized by addition of H₂O₂ to provide the corresponding carboxylic acid, compound 109.

Methods for converting the aldehyde to amines via reductive amination (e.g., using an amine and NaBH₃CN under mildly acidic conditions, or through formation of an oxime followed by catalytic hydrogenation) are well known in the art. Similarly well known are methods for converting the carboxylic acid into esters or amides such as compound 110 (e.g., through activation using a carbodiimide reagent in the presence of an alcohol or an amine). Diamines in either procedure are used to produce dimeric macrolides such as compounds 111 and 112. (See Figure 6H)

### Example 17

### Diketide thioester Synthesis: (2S,3R)-3-hydroxy-2-methyl-4-pentenoic acid N-acetylcysteamine thioester

All diketide thioesters were synthesized by a common procedure. Illustrated here is the synthesis of (2S,3R)-3-hydroxy-2-methyl-4-pentenoic acid N-acetylcysteamine thioester. Enantioselective syn-aldol condensations were performed according to the procedure of D.A. Evans et al., J Am Chem Soc (1992) 114:9434-9453. Subsequent manipulations followed the general procedures of D.E. Cane et al., J Antibiotics (1995) 48: 647-651.

The synthesis of [4S,3(2S,3R)]-4-benzyl-3-(3-hydroxy-2-methyl-4-pentenoyl)-2-oxazolidinone by aldol condensation between (4S)-N-propionyl-4-benzyl-2-oxazolidinone (1.17 g, 5.0 mmol) and acrolein (0.4 mL, 11 mmol) was performed as described by D.A. Evans et al., J Am Chem Soc (1992) 114:9434-9453, yielding 0.72 g of the adduct (50% yield) after chromatography on SiO2(2:1 hexane/ethyl acetate).

The aldol adduct was treated with t-butyldimethylsilyl trifluoromethanesulfonate (0.63 mL, 2.7 mmol) and 2,6-lutidine (0.35 mL, 3 mmol) in THF at 0°C, yielding the O-silyl ether in quantitative yield after chromatography (4:1 hexane/ethyl acetate).

A solution of the O-silyl ether in 20 mL of THF was cooled on ice, and 2.8 mL of water was added followed by 0.61 mL of 50% H₂O₂. After 10 min, a solution of 215 mg of LiOH*H₂O in 2 mL of water was added. The reaction was monitored by TLC, which revealed completion after 1 hour. A solution of 1.25 g of sodium sulfite in 8 mL of water was added, and volatiles were removed by rotary evaporation under reduced pressure. The resulting aqueous mixture was extracted three times with 20 mL portions of CH₂Cl₂, then acidified to pH 2 using 6N HCl and extracted 3 times with 50 mL portions of ethyl acetate. The ethyl acetate extracts were combined, washed with brine, dried over Na₂SO₄, filtered, and evaporated to provide the product acid as a colorless oil, 470 mg (70%).

The acid was dissolved in 10 mL of anhydrous dimethylformamide and cooled on ice. After addition of diphenylphosphorylazide (1.25 mL) and triethylamine (1.06 mL), the mixture was stirred for 2 hrs on ice.

N-acetylcysteamine (1.5 mL) was added, and the mixture was stirred overnight at room temperature. After dilution with water, the mixture was extracted 3 times with ethyl acetate. The extracts were combined, washed with brine, dried over Na₂SO₄, filtered, and evaporated to provide the crude O-silyl thioester. Chromatography (1:1 hexane/ethyl acetate) provided pure product (460 mg, 70%).

The O-silyl thioester (400 mg) was dissolved in 25 mL of acetonitrile, and 5 mL of water was added followed by 2 mL of 48% HF. After 2 hours, an additional 2 mL of 48% HF was added. After a total of 3.5 hours, the reaction was stopped by addition of sat. NaHCO₃ to neutral pH. The product was extracted with 3 portions of ethyl acetate, and the combined extracts were washed with brine, dried over Na₂SO₄, filtered, and evaporated to provide the desilylated thioester. Chromatography (Ethyl acetate) gave 150 mg (56%) of pure (2S,3R)-3-hydroxy-2-methyl-4-pentenoic acid N-acetylcysteamine thioester, APCI-MC: [M+H]+ = 232. 1H-NMR (CDCl3): d 5.83, 1H, ddd (J=5.6,10.8,17.5); 5.33, 1H, ddd (J=1.6,1.6,16.9); 5.22, 1H, ddd (J=1.5,1.5,10.8); 4.45, 1H, m; 3.45, 2H, m; 3.04, 2H, m; 2.82, 1H, dq (J=4.3,6.8); 1.96, 3H, s; 1.22, 3H, d (J=6.8).

Other diketide thioesters were prepared by substitution of appropriate aldehydes in place of acrolein.

### Example 18

### Measurement of Antibacterial Activity

Antibacterial activity was determined using either disk diffusion assays with *Bacillus cereus* as the test organism or by measurement of minimum inhibitory concentrations (MIC) in liquid culture against sensitive and resistant strains of *Staphylococcus pneumoniae.*

## Claims

1. An isolated nucleic acid molecule that encodes a non-naturally occurring modular polyketide synthase (PKS), which nucleic acid molecule is obtainable by replacing, in a nucleic acid encoding a naturally occurring PKS,
(a) the sequence encoding at least one acyl transferase catalytic region (AT) in the nucleic acid encoding said naturally occurring PKS by a sequence encoding an AT that employs a different extender unit from that employed by the AT in the naturally occurring PKS; and
(b) the sequence encoding at least one reductive cycle domain (RCD) in the nucleic acid encoding said naturally occurring PKS by a sequence encoding an RCD that contains an additional RCD catalytic activity as compared to the RCD in the naturally occurring PKS;
wherein said RCD catalytic activity is selected from keto reductase (KR), dehydratase (DH) and enoyl reductase (ER); and wherein
(i) said naturally occurring RCD comprises no RCD catalytic activity and said additional catalytic activity is KR, or KR plus DH, or KR plus DH plus ER; or
(ii) said naturally occurring RCD comprises only KR and said added catalytic activity is DH or DH plus ER; or
(iii) said naturally occurring RCD comprises only KR plus DH and said added catalytic activity is ER.

2. The nucleic acid molecule of claim 1 wherein said naturally occurring PKS is the erythromycin PKS.

3. The nucleic acid molecule of claim 1 or 2 wherein said naturally occurring RCD comprises no RCD activity and said additional catalytic activity is KR or KR plus DH or KR plus DH plus ER.

4. The nucleic acid molecule of claim 1 or 2 wherein said naturally occurring RCD comprises only KR and said added catalytic activity is DH or DH plus ER.

5. The nucleic acid molecule of claim 1 or 2 wherein said naturally occurring RCD comprises only KR plus DH and said added catalytic activity is ER.

6. The nucleic acid molecule of any of claims 1 to 5 wherein the replaced naturally occurring AT employs methylmalonyl CoA as an extender unit and is replaced by an AT that employs malonyl CoA as an extender unit.

7. The nucleic acid molecule of claim 6 wherein no more than two ATs are replaced.

8. The nucleic acid molecule of any of claims 1 to 7 wherein no more than two RCD domains are replaced by RCD domains that comprise additional RCD catalytic activities.

9. The nucleic acid molecule of any of claims 1 to 8 which further contains control sequences for expression of said PKS.

10. A recombinant host cell modified to contain the nucleic acid molecule of any of claims 1 to 9.

11. A method to prepare a modified polyketide synthase which method comprises culturing the cells of claim 10.

12. A method to prepare a polyketide which method comprises culturing the cells of claim 10 in the presences of endogenous or added starter and extender units.

## Patentansprüche

1. Isoliertes Nukleinsäuremolekül, das für eine nicht natürlich vorkommende modulare Polyketidsynthase (PKS) codiert, wobei dieses Nukleinsäuremolekül dadurch erhältlich ist, dass man in einer Nukleinsäure, die für eine natürlich vorkommende PKS codiert, Folgendes ersetzt:
(a) die Sequenz, die für mindestens eine acyltransferasekatalysierende Region (AT) in der Nukleinsäure, die für die natürlich vorkommende PKS codiert, codiert, durch eine Sequenz, die für eine AT codiert, bei der eine unterschiedliche Extender-Einheit im Vergleich zu derjenigen eingesetzt wird, die bei der AT in der natürlich vorkommenden PKS eingesetzt wird; und
(b) die Sequenz, die für mindestens eine reduktive Cyclusdomäne (RCD) in der Nukleinsäure, die für die natürlich vorkommende PKS codiert, codiert, durch eine Sequenz, die für eine RCD codiert, die eine zusätzliche RCD-Katalyseaktivität im Vergleich zu der RCD in der natürlich vorkommenden PKS enthält;
wobei die RCD-Katalyseaktivität aus der Reihe Ketoreduktase (KR), Dehydratase (DH) und Enoylreduktase (ER) ausgewählt ist; und wobei
(i) die natürlich vorkommende RCD keine RCD-Katalyseaktivität umfasst und es sich bei der zusätzlichen Katalyseaktivität um KR oder um KR plus DH oder um KR plus DH plus ER handelt; oder
(ii) die natürlich vorkommende RCD nur KR umfasst und es sich bei der hinzugefügten Katalyseaktivität um DH oder um DH plus ER handelt; oder
(iii) die natürlich vorkommende RCD nur KR plus DH umfasst und es sich bei der hinzugefügten Katalyseaktivität um ER handelt.

2. Nukleinsäuremolekül nach Anspruch 1, wobei es sich bei der natürlich vorkommenden PKS um die ErythromycinPKS handelt.

3. Nukleinsäuremolekül nach Anspruch 1 oder 2, wobei die natürlich vorkommende RCD keine RCD-Aktivität umfasst und es sich bei der zusätzlichen Katalyseaktivität um KR oder um KR plus DH oder um KR plus DH plus ER handelt.

4. Nukleinsäuremolekül nach Anspruch 1 oder 2, wobei die natürlich vorkommende RCD nur KR umfasst und es sich bei der hinzugefügten Katalyseaktivität um DH oder DH plus ER handelt.

5. Nukleinsäuremolekül nach Anspruch 1 oder 2, wobei die natürlich vorkommende RCD nur KR plus DH umfasst und es sich bei der hinzugefügten Katalyseaktivität um ER handelt.

6. Nukleinsäuremolekül nach einem der Ansprüche 1 bis 5, wobei bei der ersetzten natürlich vorkommenden AT Methylmalonyl CoA als Extender-Einheit eingesetzt wird und diese durch eine AT, bei der Malonyl-CoA als Extender-Einheit eingesetzt wird, ersetzt wird.

7. Nukleinsäuremolekül nach Anspruch 6, wobei nicht mehr als 2 ATs ersetzt werden.

8. Nukleinsäuremolekül nach einem der Ansprüche 1 bis 7, wobei nicht mehr als zwei RCD-Domänen durch RCD-Domänen, die zusätzliche RCD-Katalyseaktivitäten aufweisen, ersetzt werden.

9. Nukleinsäuremolekül nach einem der Ansprüche 1 bis 8, das weiterhin Kontrollsequenzen für die Expression der PKS enthält.

10. Rekombinante Wirtszelle, die dahingehend modifiziert ist, dass sie das Nukleinsäuremolekül nach einem der Ansprüche 1 bis 9 enthält.

11. Verfahren zur Herstellung einer modifizierten Polyketidsynthase, das das Züchten der Zellen nach Anspruch 10 umfasst.

12. Verfahren zur Herstellung eines Polyketids, wobei das Verfahren das Züchten der Zellen nach Anspruch 10 in Gegenwart von endogenen oder hinzugefügten Starter- und Extender-Einheiten umfasst.

## Revendications

1. Molécule d'acide nucléique isolé qui code pour une polycétide synthase (PKS) modulaire n'existant pas naturellement, molécule d'acide nucléique qui peut être obtenue en remplaçant, dans un acide nucléique codant pour une PKS existant naturellement,
(a) la séquence codant pour au moins une région catalytique d'acyl transférase (AT), dans l'acide nucléique qui code pour ladite PKS existant naturellement, par une séquence codant pour une AT qui emploie une unité d'extension différente de celle employée par l'AT dans la PKS existant naturellement ; et
(b) la séquence codant pour au moins un domaine de cycle réducteur (RCD), dans l'acide nucléique qui code pour ladite PKS existant naturellement, par une séquence codant pour un RCD qui contient une activité catalytique de RCD supplémentaire, en comparaison avec le RCD dans la PKS existant naturellement ;
dans laquelle ladite activité catalytique de RCD est choisie parmi une céto réductase (KR), une déshydratase (DH) et une énoyl réductase (ER) ; et dans laquelle
(i) ledit RCD existant naturellement ne comprend aucune activité catalytique de RCD et ladite activité catalytique supplémentaire est une KR, ou une KR plus une DH, ou une KR plus une DH plus une ER ; ou
(ii) ledit RCD existant naturellement comprend seulement une KR et ladite activité catalytique ajoutée est une DH, ou une DH plus une ER ; ou
(iii) ledit RCD existant naturellement comprend seulement une KR plus une DH et ladite activité catalytique ajoutée est une ER.

2. Molécule d'acide nucléique selon la revendication 1, dans laquelle ladite PKS existant naturellement est la PKS à érythromycine.

3. Molécule d'acide nucléique selon la revendication 1 ou la 2, dans laquelle ledit RCD existant naturellement ne comprend aucune activité de RCD et ladite activité catalytique supplémentaire est une KR, ou une KR plus une DH, ou une KR plus une DH plus une ER.

4. Molécule d'acide nucléique selon la revendication 1 ou la 2, dans laquelle ledit RCD existant naturellement comprend seulement une KR et ladite activité catalytique ajoutée est une DH, ou une DH plus une ER.

5. Molécule d'acide nucléique selon la revendication 1 ou la 2, dans laquelle ledit RCD existant naturellement comprend seulement une KR plus une DH et ladite activité catalytique ajoutée est une ER.

6. Molécule d'acide nucléique selon l'une quelconque des revendications 1 à 5, dans laquelle l'AT existant naturellement remplacée emploie le méthylmalonyl CoA comme unité d'extension et elle est remplacée par une AT qui emploie le malonyl CoA comme unité d'extension.

7. Molécule d'acide nucléique selon la revendication 6, dans laquelle pas plus de deux AT sont remplacées.

8. Molécule d'acide nucléique selon l'une quelconque des revendications 1 à 7, dans laquelle pas plus de deux domaines RCD sont remplacés par des domaines RCD qui comprennent des activités catalytiques de RCD supplémentaires.

9. Molécule d'acide nucléique, selon l'une quelconque des revendications 1 à 8, qui contient en outre des séquences de contrôle pour l'expression de ladite PKS.

10. Cellule hôte recombinante modifiée pour contenir la molécule d'acide nucléique selon l'une quelconque des revendications 1 à 9.

11. Procédé destiné à préparer une polycétide synthase modifiée, procédé qui comprend la culture des cellules selon la revendication 10.

12. Procédé destiné à préparer un polycétide, procédé qui comprend la culture des cellules, selon la revendication 10, en présence d'unités de démarrage et d'extension endogènes ou ajoutées.
